# EUROPÄISCHE PATENTSCHRIFT

(11) **EP 3 443 413 B1**
(45) Veröffentlichungstag und Bekanntmachung des Hinweises auf die Patenterteilung: **28.10.2020**
(21) Anmeldenummer: 17717348.1
(22) Anmeldetag: 05.04.2017
(51) Int. Cl.: G03B 42/08, G03B 42/02, A61B 6/00

(54) **VORRICHTUNG ZUR OPTISCHEN AUFNAHME EINES SCHIRMS**
DEVICE FOR OPTICALLY RECEIVING A SCREEN
DISPOSITIF DE PRISE D'IMAGE OPTIQUE D'UN ÉCRAN

(30) Priorität: 15.04.2016 DE 102016206444
(43) Veröffentlichungstag der Anmeldung: 20.02.2019
(73) Patentinhaber: Fraunhofer-Gesellschaft zur Förderung der angewandten Forschung e.V., 80686 München (DE)
(72) Erfinder: BEHRENDT, Rolf, 91077 Dormitz (DE); GRÖGER, Wolfgang, 91341 Röttenbach (DE); SCHMITT, Peter, 91058 Erlangen (DE); JOBST, Andreas, 91058 Erlangen (DE); KOSTKA, Günther, 91056 Erlangen (DE)
(74) Vertreter: Schlenker, Julian
(86) Internationale Anmeldenummer: PCT/EP2017/058103
(87) Internationale Veröffentlichungsnummer: WO 2017/178300

(56) Entgegenhaltungen:
- WO-A1-2014/000810
- DE-A1-102013 104 835
- JP-A- 2010 253 049

## Beschreibung

Ausführungsbeispiele der vorliegenden Erfindung beziehen sich auf eine Bildaufnahmevorrichtung, und im speziellen, auf eine Bildaufnahmevorrichtung zur optischen Aufnahme eines Schirms (beispielsweise ein Szintillatorschirms) mit mehreren Kameras. Manche Ausführungsbeispiele beziehen sich auf eine strahlungsgeschützte Röntgenkamera, die in der digitalen Radioskopie beispielsweise für eine Qualitätskontrolle von Produkten eingesetzt werden kann.

In der industriellen und medizinischen Radioskopie werden zur Zeit vorzugsweise sogenannte Flat-Panel-Detektoren eingesetzt. Bei diesen wird die Röntgenstrahlung in der Regel über einen Szintillatorschirm in sichtbares Licht gewandelt und dieses über eine in Strahlrichtung direkt hinter dem Szintillatorschirm angeordnete Halbleiterschicht (amorphes oder kristallines Silizium) nachgewiesen und in ein Bild umgesetzt. Die Effizienz eines Szintillatorschirms hängt unter anderem von der eingestellten Energie der Röntgenquanten ab. Je höher die Energie der Röntgenstrahlung ist, desto weniger Röntgenquanten werden im Szintillator absorbiert und tragen zum Bild bei. Die nicht absorbierten Röntgenquanten können von der darunter liegenden Halbleiterschicht absorbiert werden, wodurch diese geschädigt wird. Diese Strahlenschäden führen bei entsprechender Dosis letztlich zum Ausfall des Detektors.

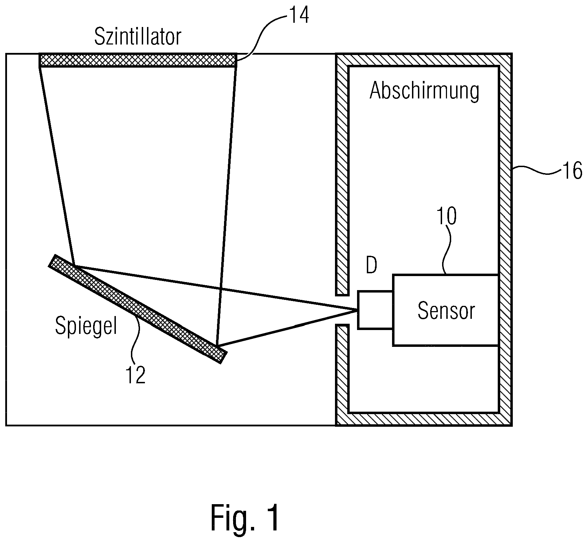

Des Weiteren sind Röntgendetektoren im Einsatz, bei denen die Röntgenstrahlung zunächst ebenfalls durch einen Szintillator in sichtbares Licht gewandelt wird, dieses jedoch anschließend mittels Bildverstärker oder einer optischen Abbildung über ein Objektiv z.B. auf CCD- oder CMOS-Kameras direkt oder mittels eines Spiegels abgebildet wird, wie dies in Fig. 1 gezeigt ist.

Im Detail ist in Fig. 1 eine Spiegel-/Kameraanordnung zu erkennen, bei der eine Kamera 10 über einen Spiegel 12 einen Szintillator 14 aufnimmt. Die Kamera 10 ist dabei in Bezug auf den Szintillator 14 so angeordnet, dass eine Aufnahmerichtung der Kamera (Sensor) 10 parallel zu einer Szintillatorebene verläuft. Die Kamera 10 ist dabei von einer Abschirmung 16 umhüllt, die in einem Bereich benachbart zu einem Objektiv der Kamera 10 eine Öffnung aufweist.

Mit anderen Worten, Fig. 1 zeigt eine einfache einreihige Spiegel- und Kameraanordnung nach dem Stand der Technik. Senkrecht zur Zeichenebene können beliebig viele Kameras 10 in Reihen angeordnet sein.

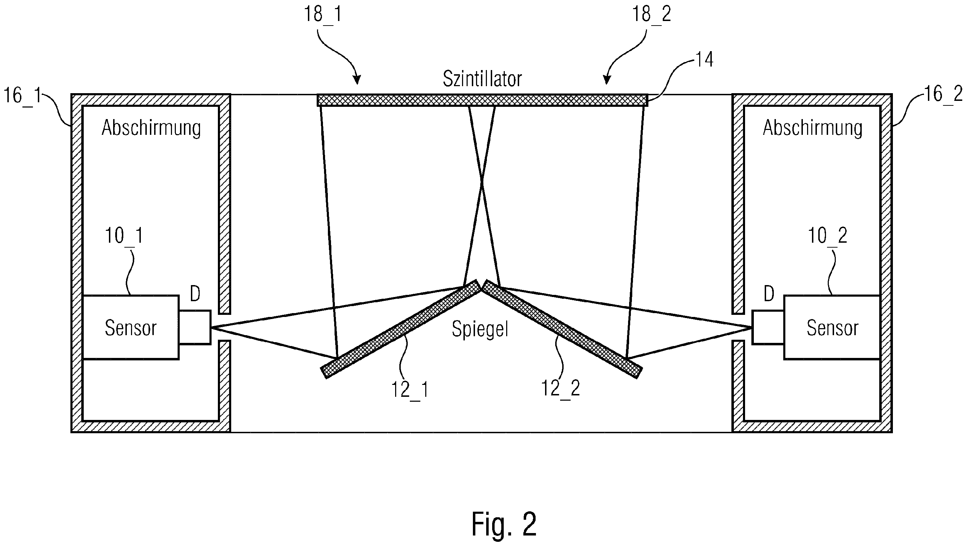

Die EP 0 862 748 beschreibt eine Anordnung, bei der das vom Szintillator ausgehende sichtbare Licht über eine V-förmige Spiegelanordnung umgelenkt wird, so dass der optische Strahlengang hinter dem Spiegel im Wesentlichen parallel zum Szintillatorschirm liegt. Hierdurch können die strahlungsempfindlichen Kameras außerhalb des Röntgen-Strahlengangs angeordnet werden und Strahlenschäden vermieden werden. Fig. 2 zeigt ein Beispiel für eine Anordnung nach dem Stand der Technik, wobei die V-förmige Spiegelanordnung von EP 0 862 748 dargestellt ist.

Bei der in Fig. 2 gezeigten Spiegel-/Kameraanordnung nehmen zwei Kameras 10_1 und 10_2 über jeweils einen Spiegel 12_1 und 12_2 einen Abschnitt 18_1 und 18_2 eines Szintillators 14 auf. Die Kameras 10_1 und 10_2 sind dabei in Bezug auf den Szintillator 14 so angeordnet, dass Aufnahmerichtungen der Kameras 10_1 und 10_2 parallel zu einer Szintillatorebene verlaufen. Ferner sind die Kameras 10_1 und 10_2 jeweils von einer Abschirmung 16_1 und 16_2 umhüllt, die in Bereichen benachbart zu Objektiven der jeweiligen Kameras 10_1 und 10_2 Öffnungen aufweisen.

Fig. 2 zeigt somit eine V-förmige Spiegelanordnung gemäß der EP 0 862 748 bzw. eine zweireihige Spiegel- und Kameraanordnung nach dem Stand der Technik. Senkrecht zur Zeichenebene können beliebig viele Kameras in Reihen angeordnet sein.

Die DE 103 01 941 beschreibt eine Anordnung, bei der das vom Szintillator ausgehende sichtbare Licht über zwei parallel zueinander angeordnete Spiegel umgelenkt wird und so zur optischen Kamera gelangt. Durch eine periodische Aneinanderreihung dieser parallelen Spiegelanordnung ist es möglich, mit einer Vielzahl von optischen Kameras eine beliebig große Szintillatorfläche abzubilden.

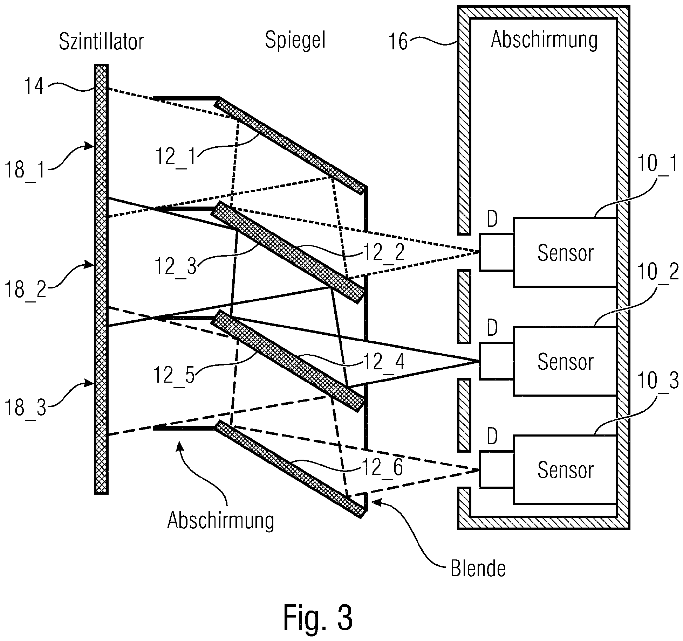

Fig. 3 zeigt ein Beispiel für eine solche Spiegel-/Kameraanordnung. Drei Kameras 10_1 bis 10_3 nehmen über jeweils zwei Spiegel 12_1 bis 12_6 einen Abschnitt 18_1 bis 18_3 des Szintillators auf. Die Kameras 10_1 bis 10_3 sind dabei in Bezug auf den Szintillator 14 so angeordnet, dass die Aufnahmerichtungen der Kameras 10_1 bis 10_3 senkrecht auf einer Szintillatorebene stehen. Die drei Kameras 10_1 bis 10_3 sind ferner von einer Abschirmung 16 umhüllt, die in Bereichen benachbart zu Objektiven der jeweiligen Kameras 10_1 bis 10_3 Öffnungen aufweist.

Fig. 3 zeigt somit eine Parallel-Spiegelanordnung gemäß Patent der DE 103 01 941 bzw. eine Spiegel- und Kameraanordnung nach dem Stand der Technik. Dabei können senkrecht zur Zeichenebene und in vertikaler Richtung beliebig viele Kameras in Reihen angeordnet sein.

Um die optischen Kameras 10_1 bis 10_3 vor Röntgenstrahlung zu schützen, die durch den Szintillator 14 oder durch die Spiegel 12_1 bis 12_6 nicht absorbiert wird, befindet sich zwischen jeweils benachbarten Spiegeln ein Absorbermaterial (z.B. Blei). Weiterhin wird im optischen Strahlengang ein spezielles Glas angeordnet, das für sichtbares Licht transparent ist, Röntgenstrahlung jedoch stark absorbiert (z.B. Bleiglas). Zwischen den Gläsern befindet sich ein weiterer Absorber. Aufgrund der räumlichen Randbedingungen kann der Abstand zwischen jeweils benachbarten Spiegeln nicht beliebig groß gewählt werden, wodurch die maximale Dicke der jeweils zwischen den Spiegeln eingesetzten Bleiabsorber begrenzt ist, so dass die Strahlenabschirmwirkung bei sehr hohen Röntgenenergien (z.B. größer als 220 keV, oder größer als 450 keV) nicht mehr gegeben ist.

Der o.g. Nachteil der im Dokument DE 103 01 941 beschriebenen paarweisen Anordnung von Spiegel bzgl. der Abschirmung gegen durch den Szintillator dringenden Röntgenstrahlung besteht also darin, dass die Strahlungsabschirmwirkung im Wesentlichen durch das zwischen den beiden benachbarten Spiegeln positionierte Absorbermaterial erreicht wird. D.h. für die Einsatzfähigkeit einer kachelbaren Anordnung von optischen Kameras zur Abbildung eines Szintillatorschirms bei höheren Energie besteht also die Notwendigkeit der Bereitstellung einer Vorrichtung, bei der die Kameras ebenfalls beliebig gekachelt werden können, bei der aber die maximale Dicke des Absorbermaterials deutlich höher sein kann.

Die JP 2010 253049 A offenbart ein Radiotherapiegerät und Strahlenfluoroskop. Szintillatorplatten erzeugen eine Fluoreszenz gemäß einer vorbestimmten Absorptionsenergieverteilung durch therapeutische Strahlung. Eine Lichtabschirmplatte schirmt die von den Szintillatorplatten erzeugte Fluoreszenz in jeweils unterschiedliche Richtungen ab. Die Kameras nehmen jeweils ein Bild der von den Szintillatorplatten erzeugten Fluoreszenz aus jeweils nicht abgeschirmten Richtungen auf. Dann erzeugt eine Steuerung unter Verwendung des von den Kameras aufgenommenen Bildes Bilder, die durch jedes biologische Gewebe getrennt sind, das in dem betroffenen Bereich enthalten ist, der ein der Therapie unterworfener Bereich ist.

Der vorliegenden Erfindung liegt somit die Aufgabe zugrunde, ein Konzept zu schaffen, welches eine bessere Abschirmung der Kameras bei kompakter Bauweise ermöglicht.

Diese Aufgabe wird durch die unabhängigen Patentansprüche gelöst.

Ausführungsbeispiele schaffen eine Bildaufnahmevorrichtung mit einem Schirm, einer Mehrzahl von Spiegeln und einer Mehrzahl von Kameras. Die Mehrzahl von Spiegeln und die Mehrzahl von Kameras ist so angeordnet, dass die Mehrzahl von Kameras über jeweils einen der Mehrzahl von Spiegeln jeweils einen Abschnitt des Schirms aufnimmt, wobei die Mehrzahl von Kameras schräg in Bezug auf den Schirm angeordnet ist.

Der vorliegenden Erfindung liegt die Idee zugrunde, dass bei einer gekachelten Anordnung von Kameras eine Abschirmung der Kameras verbessert werden kann (z.B. durch ein dickeres Absorbermaterial zwischen Schirm und Kamera) ohne eine Baugröße der Bildaufnahmevorrichtung im Wesentlichen zu vergrößern, wenn die Mehrzahl von Kameras schräg in Bezug auf den Schirm angeordnet ist, und jeweils einen Abschnitt des Schirms über nur einen Spiegel aufnehmen.

Weitere Ausführungsbeispiele schaffen ein Verfahren. Das Verfahren umfasst einen Schritt des Aufnehmens des Schirms über eine Mehrzahl von Spiegeln mit einer Mehrzahl von Kameras, wobei die Mehrzahl von Spiegeln und die Mehrzahl von Kameras so angeordnet ist, dass die Mehrzahl von Kameras über jeweils einen der Mehrzahl von Spiegeln einen Abschnitt des Schirms aufnimmt, und wobei die Mehrzahl von Kameras schräg in Bezug auf den Schirm angeordnet ist.

Vorteilhafte Weiterbildungen finden sich in den abhängigen Patentansprüchen.

Bei Ausführungsbeispielen kann die Mehrzahl von Kameras in Bezug auf den Schirm schräg angeordnet sein, d.h. Aufnahmerichtungen der Kameras verlaufen weder parallel noch senkrecht zu einer Ebene des Schirms (Schirmebene) bzw. einer durch den Schirm aufgespannte Ebene. Beispielsweise können die Winkel zwischen den Aufnahmerichtungen der Mehrzahl von Kameras und der Schirmebene jeweils im Bereich zwischen 5° und 85° (oder 10° und 80°, oder 15° und 75°, oder 20° und 70°, oder 25° und 65°, oder 30° und 60°) liegen.

Bei Ausführungsbeispielen kann die Mehrzahl von Kameras so angeordnet sein, dass die Aufnahmerichtungen der Mehrzahl von Kameras parallel zueinander verlaufen.

Ferner kann die Mehrzahl von Spiegeln gekippt in Bezug auf den Schirm angeordnet sein, d.h. Spiegelebenen bzw. Ebenen der Mehrzahl von Spiegeln verlaufen weder parallel noch senkrecht zu einer Ebene des Schirms (Schirmebene) bzw. einer durch den Schirm aufgespannte Ebene.

Bei Ausführungsbeispielen kann zumindest eine der Mehrzahl von Kameras (direkt oder unmittelbar) hinter dem Schirm angeordnet sein, d.h. in Richtung einer auf den Schirm treffenden Strahlung. Wenn die Strahlung senkrecht auf den Schirm trifft, dann kann die Mehrzahl von Kameras so angeordnet sein, dass eine Projektion des Schirms entlang einer Schirmnormalen auf die zumindest eine der Mehrzahl von Kameras trifft.

Bei Ausführungsbeispielen kann die Vorrichtung zumindest ein Abschirmelement aufweisen, dass zwischen dem Schirm und einer der Mehrzahl von Kameras angeordnet ist, so dass die zumindest eine der Mehrzahl von Kameras in Bezug auf den Schirm vollständig hinter dem Abschirmelement angeordnet ist. Beispielsweise kann das zumindest eine Abschirmelement (Absorber) in Richtung der auf den Schirm treffenden Strahlung zwischen Schirm und der zumindest einen der Mehrzahl von Kameras angeordnet sein, so dass die zumindest eine der Mehrzahl von Kameras vor einer durch den Schirm hindurchtretenden Strahlung geschützt ist.

Das zumindest eine Abschirmelement kann dabei außerhalb eines optischen Strahlengangs angeordnet sein, der zwischen der einen der Mehrzahl von Kameras und dem jeweiligen Abschnitt des Schirms über den jeweiligen der Mehrzahl von Spiegeln verläuft. Die Mehrzahl von Kameras, die Mehrzahl von Spiegeln und das zumindest eine Abschirmelement können also so angeordnet sein, dass der optische Strahlengang der zumindest einen der Mehrzahl von Kameras, die hinter dem zumindest einen Abschirmelement angeordnet ist, an dem zumindest einem Abschirmelement vorbei verläuft.

Bei Ausführungsbeispielen kann die Bildaufnahmevorrichtung eine Abschirmung aufweisen, die die Mehrzahl von Spiegeln und die Mehrzahl von Kameras einschließt und an Seiten des Schirms angrenzt.

Die Abschirmung kann dabei so geformt sein, dass ein Abschnitt der Abschirmung zwischen Schirm und einer der Mehrzahl von Kameras angeordnet ist, so dass die eine Kamera in Bezug auf den Schirm vollständig hinter dem Abschnitt der Abschirmung angeordnet ist. Zumindest eine andere der Mehrzahl von Kameras kann dabei hinter dem zumindest einen Abschirmelement angeordnet sein. Der Abschnitt der Abschirmung und das zumindest eine Abschirmelement können dabei die gleiche oder eine ähnliche Form aufweisen. Ferner können der Abschnitt der Abschirmung und das zumindest eine Abschirmelement gleich oder ähnlich (in Bezug zueinander bzw. in Bezug auf den Schirm oder die jeweilige der Mehrzahl von Kameras) angeordnet sein.

Bei Ausführungsbeispielen kann die Bildaufnahmevorrichtung eine Mehrzahl von Strahlenschutzgläsern aufweisen, die zwischen der Mehrzahl von Spiegeln und der Mehrzahl von Kameras angeordnet sind. Die Mehrzahl von Strahlenschutzgläsern kann demnach in den optischen Strahlengängen zwischen der Mehrzahl von Spiegeln und der Mehrzahl von Kameras liegen.

Bei Ausführungsbeispielen kann die Mehrzahl von Kameras den jeweiligen Abschnitt des Schirms jeweils über genau einen der Mehrzahl von Spiegeln aufnehmen. Mit anderen Worten, die eine Anzahl der Spiegel kann gleich einer Anzahl der Kameras sein.

Bei Ausführungsbeispielen kann der Schirm ein Szintillator sein. Ein Szintillator ist dazu geeignet Röntgenstrahlung in sichtbares Licht umzuwandeln. Im Detail werden die Moleküle vom Szintillator, wenn Röntgenstrahlung (oder andere energiereiche Strahlung) auf den Szintillator trifft, durch Stoßprozesse angeregt, wobei der Szintillator diese Anregungsenergie in Form von sichtbarem Licht wieder abgibt.

Bei Ausführungsbeispielen können die Mehrzahl von Spiegeln und die Mehrzahl von Kameras so angeordnet sein, dass die Mehrzahl von Kameras sich zumindest teilweise unterscheidende Abschnitte des Schirms aufnehmen. Die Mehrzahl von Kameras kann also so angeordnet sein, dass diese aneinander angrenzende bzw. sich teilweise überlappende Abschnitte des Schirms aufnehmen.

Bei Ausführungsbeispielen kann die Mehrzahl von Kameras Scheimpflug-Optiken aufweisen, wobei die Mehrzahl von Spiegeln und die Mehrzahl von Kameras so angeordnet sind, dass die Mehrzahl von Kameras die jeweiligen Abschnitte des Schirms über die jeweiligen Spiegel jeweils unter Scheimpflug-Bedingungen aufnimmt. Die Scheimpflug-Bedingung wird in der Regel angewandt zur optischen Abbildung von zueinander verkippten Objekt- und Bildebenen mittels Objektiv. Die Scheimpflug-Regel besagt, dass sich die Objekt-, die Objektiv- und die Bildebene in einer gemeinsamen Gerade schneiden müssen, um eine scharfe Abbildung der Objektebene auf eine dazu schräg stehenden Bildebene zu erreichen.

Ausführungsbeispiele der vorliegenden Erfindung schaffen gegenüber der EP 0 862 748 und DE 103 01 941 verbesserte Methoden/Konzepte zur Aufteilung und Abbildung des Szintillatorschirms mit einer prinzipiell beliebigen Anzahl optischer Kameras.

Gegenüber der EP 0 862 748 besteht der Vorteil von Ausführungsbeispielen der vorliegenden Erfindung darin, dass ohne Vergrößerung der Bautiefe in Richtung der eintreffenden Röntgenstrahlung eine beliebig große Szintillatorfläche auf eine entsprechend große Anzahl optischer Kameras aufgeteilt werden kann und die Ausmaße der Röntgenkamera senkrecht zur Strahlrichtung im Wesentlichen der Größe des abzubildenden Szintillatorschirms entsprechen, während bei der in der EP 0 862 748 beschriebenen Methode die Ausmaße der Röntgenkamera deutlich größer sind als die Maße des Szintillatorschirms, da dort die optischen Kameras seitlich angeordnet werden.

Der Vorteil von Ausführungsbeispielen der vorliegenden Erfindung gegenüber der DE 103 01 941 besteht darin, dass aufgrund der speziellen optischen Abbildungsgeometrie aus Spiegel- und Kameraanordnung ein Absorber mit einer wesentlich größeren Dicke zum Schutz vor Röntgenstrahlung eingesetzt werden kann, so dass der Röntgendetektor für einen signifikant höheren Röntgenenergiebereich einsetzbar ist.

Ausführungsbeispiele der vorliegenden Erfindung werden bezugnehmend auf die beiliegenden Figuren näher erläutert. Es zeigen:
- Fig. 1: eine bekannte Spiegel-/Kameraanordnung bei der eine Kamera über einen Spiegel einen Szintillator aufnimmt;
- Fig. 2: eine bekannte Spiegel-/Kameraanordnung bei der zwei Kameras über jeweils einen Spiegel einen Abschnitt eines Szintillators aufnehmen;
- Fig. 3: eine bekannte Spiegel-/Kameraanordnung bei der drei Kameras über jeweils zwei Spiegel einen Abschnitt eines Szintillators aufnehmen;
- Fig. 4: eine schematische Ansicht einer Bildaufnahmevorrichtung, gemäß einem Ausführungsbeispiel der vorliegenden Erfindung;
- Fig. 5: eine schematische Ansicht einer Bildaufnahmevorrichtung mit drei schräg angeordneten Kameras, die jeweils über genau einen Spiegel einen Abschnitt des Szintillators aufnehmen, gemäß einem Ausführungsbeispiel der vorliegenden Erfindung;
- Fig. 6: eine schematische Ansicht einer Bildaufnahmevorrichtung mit drei Kameras, die unter Scheimpflug-Bedingungen über jeweils einen Spiegel einen Abschnitt des Szintillators aufnehmen, gemäß einem Ausführungsbeispiel der vorliegenden Erfindung;
- Fig. 7: eine schematische Ansicht einer Bildaufnahmevorrichtung, gemäß einem Ausführungsbeispiel der vorliegenden Erfindung; und
- Fig. 8: ein Flussdiagramm eines Verfahrens zum Aufnehmen eines Schirms, gemäß einem Ausführungsbeispiel.

In der nachfolgenden Beschreibung der Ausführungsbeispiele der Erfindung werden in den Figuren gleiche oder gleichwirkende Elemente mit den gleichen Bezugszeichen versehen, so dass deren Beschreibung in den unterschiedlichen Ausführungsbeispielen untereinander austauschbar ist.

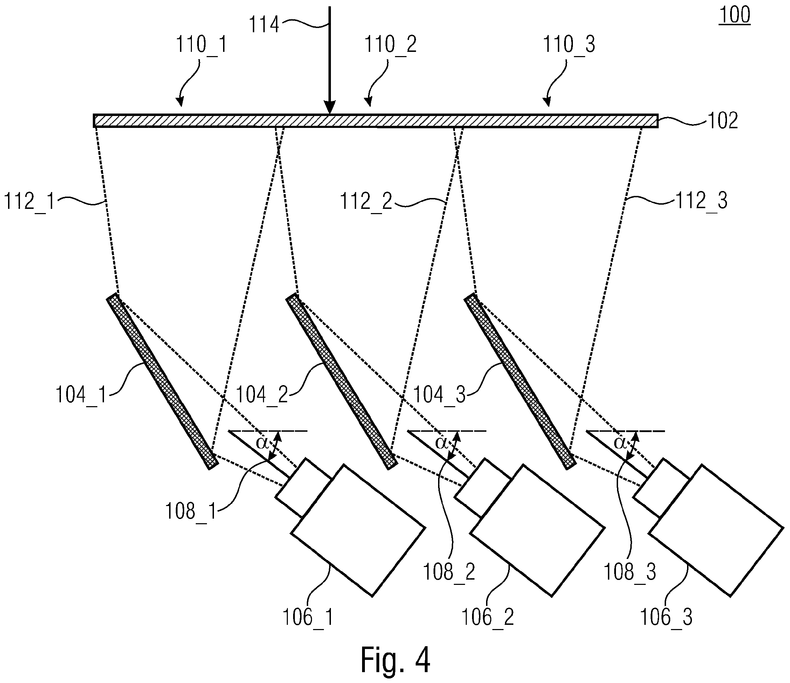

Fig. 4 zeigt eine schematische Ansicht einer Bildaufnahmevorrichtung 100, gemäß einem Ausführungsbeispiel der vorliegenden Erfindung. Die Bildaufnahmevorrichtung 100 weist einen Schirm 102, eine Mehrzahl von Spiegeln 104_1 bis 104_n, und eine Mehrzahl von Kameras 106_1 bis 106_n auf (n kann eine natürliche Zahl größer gleich zwei sein, n ≥ 2). Die Mehrzahl von Spiegeln 104_1 bis 104_n und die Mehrzahl von Kameras 106_1 bis 106_n sind dabei so angeordnet, dass die Mehrzahl von Kameras 106_1 bis 106_n über jeweils einen der Mehrzahl von Spiegeln 104_1 bis 104_n einen Abschnitt 108_1 bis 108_n des Schirms aufnimmt. Die Mehrzahl von Kameras 106_1 bis 106_n ist dabei schräg in Bezug auf den Schirm 102 angeordnet.

Wie in Fig. 4 zu erkennen ist, ist die Mehrzahl von Kameras 106_1 bis 106_n in Bezug auf den Schirm 102 schräg angeordnet, d.h. Aufnahmerichtungen der Kameras 106_1 bis 106_n verlaufen weder parallel noch senkrecht zu einer Ebene des Schirms (Schirmebene) bzw. eine durch den Schirm aufgespannte Ebene.

Beispielsweise können die Winkel α zwischen den Aufnahmerichtungen (senkrecht zur Objektivebene) 108_1 bis 108_n der Mehrzahl von Kameras und der Schirmebene jeweils im Bereich zwischen 5° und 85° (oder 10° und 80°, oder 15° und 75°, oder 20° und 70°, oder 25° und 65°, oder 30° und 60°) liegen.

Die Aufnahmerichtungen 108_1 bis 108_n der Mehrzahl von Kameras 106_1 bis 106_n können parallel zueinander verlaufen, d.h. die Mehrzahl von Kameras 106_1 bis 106_n können im gleichen Winkel in Bezug auf den Schirm angeordnet sein.

Ferner kann die Mehrzahl von Spiegeln 104_1 bis 104_3 gekippt in Bezug auf den Schirm angeordnet sein, d.h. Spiegelebenen bzw. Ebenen der Mehrzahl von Spiegeln 104_1 bis 104_3 verlaufen weder parallel noch senkrecht zu einer Ebene des Schirms (Schirmebene) bzw. eine durch den Schirm aufgespannte Ebene.

Im Folgenden werden Ausführungsbeispiele der Bildaufnahmevorrichtung 100 beschrieben, bei denen die Bildaufnahmevorrichtung 100 drei Spiegel 104_1 bis 104_3 und drei Kameras 106_1 bis 106_3 aufweist. Die nachfolgende Beschreibung ist jedoch in entsprechender Weise auch auf Ausführungsbeispiele der Bildaufnahmevorrichtung 100 anwendbar, bei denen die Bildaufnahmevorrichtung n Spiegel 104_1 bis 104_n und n Kameras 106_1 bis 106_n aufweist, wobei n eine natürliche Zahl größer gleich zwei ist.

Wie in Fig. 4 zu erkennen ist, erfasst eine erste Kamera 106_1 über einen ersten Spiegel 104_1 einen ersten Abschnitt 110_1 des Schirms 102, eine zweite Kamera 106_2 über einen zweiten Spiegel 104_2 einen zweiten Abschnitt 110_2 des Schirms 102 erfasst, und eine dritte Kamera 106_3 über einen dritten Spiegel 104_3 einen dritten Abschnitt 110_3 des Schirms 102. Dementsprechend verläuft ein erster optischer Strahlengang 112_1 zwischen der ersten Kamera 106_1 und dem ersten Abschnitt 110_1 des Schirms 102 über den ersten Spiegel 104_1, ein zweiter optischer Strahlengang 112_2 zwischen der zweiten Kamera 106_2 und dem zweiten Abschnitt 110_2 des Schirms 102 über den zweiten Spiegel 104_2, und ein dritter optischer Strahlengang 112_3 zwischen der dritten Kamera 106_3 und dem dritten Abschnitt 110_3 des Schirms 102 über den dritten Spiegel 104_3.

Die drei Spiegel 104_1 bis 104_3 und die drei Kameras 106_1 bis 106_3 können so angeordnet sein, dass die drei Kameras 106_1 bis 106_3 sich zumindest teilweise unterscheidende Abschnitte 110_1 bis 110_3 des Schirms 102, d.h. aneinander angrenzende bzw. sich teilweise überlappende Abschnitte des Schirms 102, aufnehmen.

Jeder der drei Kameras 106_1 bis 106_3 kann dabei genau ein Spiegel zugeordnet sein, d.h. jede der drei Kameras 106_1 bis 106_3 nimmt den jeweiligen Abschnitt (Bereich) des Schirms 102 über nur einen Spiegel auf.

Der Schirm 102 kann ein Szintillator sein, der ausgebildet ist, um auf den Szintillator treffende Strahlung (z.B. Röntgenstrahlung) in Licht umzuwandeln, dessen Wellenlänge bzw. Wellenlängen in einem Bereich liegt, der durch die Kameras aufgenommen werden kann.

Die erste Kamera 106_1 und die zweite Kamera 106_2 können hinter dem Schirm 102 angeordnet sein, oder mit anderen Worten, hinter dem Schirm 102 in Richtung der auf den Schirm 102 treffenden Strahlung (angedeutet durch den Pfeil 114 in Fig. 4). Für den Fall, dass die Strahlung senkrecht auf den Schirm trifft, würde eine Projektion des Schirms 102 entlang einer Schirmnormalen somit auf die erste Kamera 106_1 und die zweite Kamera 106_2 treffen.

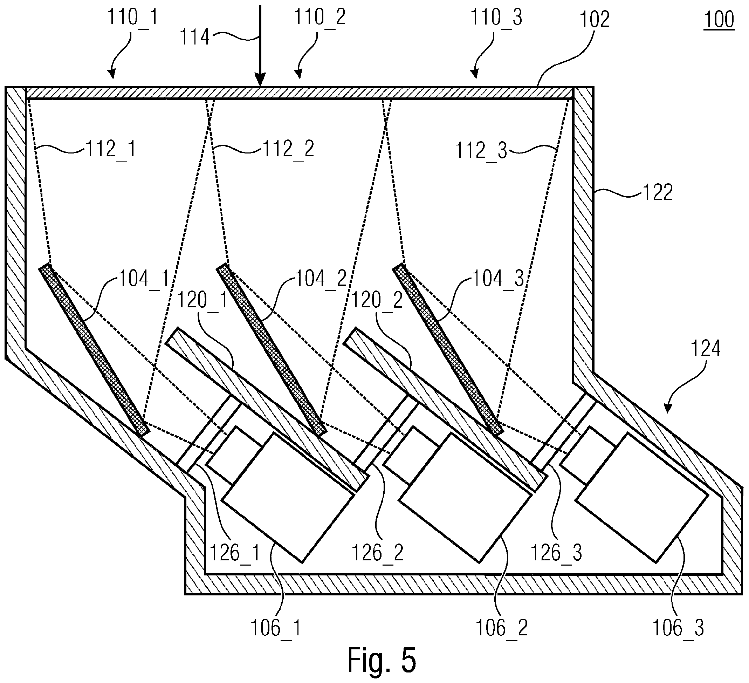

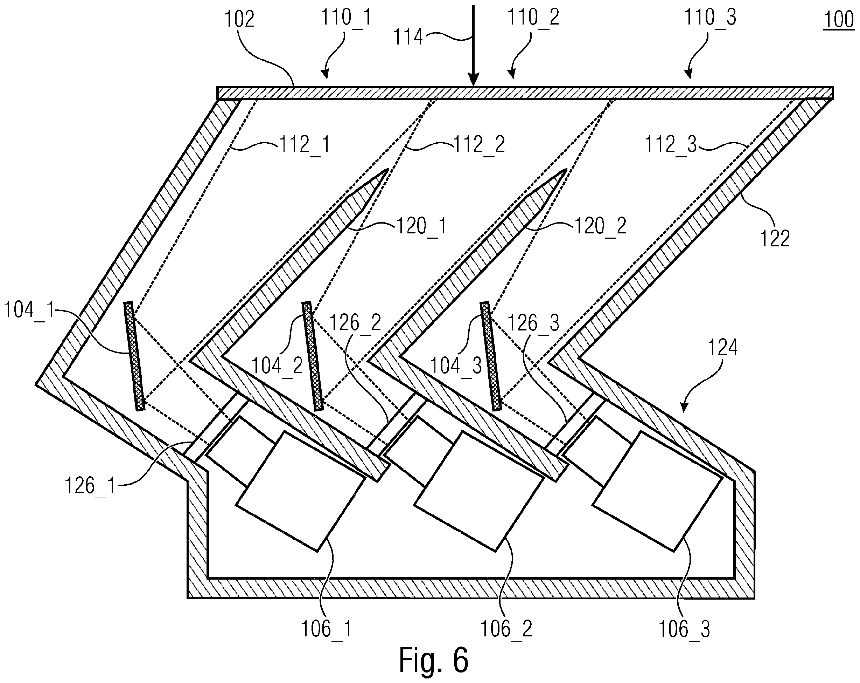

Dadurch, dass die Kameras 106_1 bis 106_3 schräg in Bezug auf den Schirm angeordnet sind und den jeweiligen Abschnitt 110_1 bis 110_3 des Schirms 102 jeweils über nur einen Spiegel 104_1 bis 104_3 aufnehmen, kann ein dickeres Absorbermaterial zwischen dem Schirm 102 und den Kameras 106_1 bis 106_3 angeordnet werden, so dass eine Abschirmung der Kameras 106_1 bis 106_3 verbessert werden kann ohne eine Baugröße der Bildaufnahmevorrichtung 100 (wesentlich) zu erhöhen, wie im Folgenden anhand der in Fig. 5 und 6 gezeigten Ausführungsbeispiele ausgeführt wird.

Fig. 5 zeigt eine schematische Ansicht einer Bildaufnahmevorrichtung 100 mit drei schräg angeordneten Kameras 106_1 bis 106_3, die jeweils über genau einen Spiegel 104_1 bis 104_3 einen Abschnitt 110_1 bis 110_3 des Szintillators 102 aufnehmen. Mit anderen Worten, Fig. 5 zeigt eine optische Abbildung, bei der die Zentralstrahlen der Kameras bzw. der Objektive senkrecht zu einer Ebene des Szintillators verlaufen. Es sei darauf hingewiesen, dass, obwohl in Fig. 5 nur drei Kameras gezeigt sind, senkrecht zur Zeichenebene und in horizontaler Richtung beliebig viele Kameras in Reihen angeordnet sein können.

Wie in Fig. 5 zu erkennen ist kann die Bildaufnahmevorrichtung 100 ein erstes Abschirmelement 120_1 und ein zweites Abschirmelement 120_2 aufweisen. Das erste Abschirmelement 120_1 kann zwischen dem Szintillator 102 und der ersten Kamera 106_1 angeordnet sein, so dass die erste Kamera 106_1 in Bezug auf den Szintillator 102 vollständig hinter dem ersten Abschirmelement 120_1 angeordnet ist. Das zweite Abschirmelement 120_2 kann zwischen dem Szintillator 102 und der zweiten Kamera 106_2 angeordnet sein, so dass die zweite Kamera 106_2 in Bezug auf den Szintillator 102 vollständig hinter dem zweiten Abschirmelement 120_2 angeordnet ist.

Beispielsweise kann das erste Abschirmelement (Absorber) 120_1 in Richtung der auf den Szintillator 102 treffenden Strahlung (angedeutet durch den Pfeil 114 in Fig. 5) zwischen Szintillator 102 und der ersten Kamera 106_1 angeordnet sein, so dass die erste Kamera 106_1 vor einer durch den Szintillator 102 hindurchtretenden Strahlung (z.B. Röntgenstrahlung) geschützt ist. Genauso kann das zweite Abschirmelement (Absorber) 120_2 in Richtung der auf den Szintillator 102 treffenden Strahlung zwischen Szintillator 102 und der zweiten Kamera 106_2 angeordnet sein, so dass die zweite Kamera 106_2 vor einer durch den Szintillator 102 hindurchtretenden Strahlung (z.B. Röntgenstrahlung) geschützt ist.

Das erste Abschirmelement 120_1 kann dabei so ausgebildet und angeordnet sein, dass dieses nicht im ersten optischen Strahlengang liegt, der zwischen der ersten Kamera 106_1 und dem ersten Abschnitt 110_1 des Szintillators 102 über den ersten Spiegel 104_1 verläuft. Der erste optische Strahlengang 112_1 verläuft somit an dem ersten Abschirmelement 120_1 vorbei bzw. um dieses herum. Genauso kann das zweite Abschirmelement 120_2 so ausgebildet und angeordnet sein, dass dieses nicht im zweiten optischen Strahlengang 112_2 liegt, der zwischen der zweiten Kamera 106_2 und dem zweiten Abschnitt 110_2 des Szintillators 102 über den zweiten Spiegel 104_1 verläuft. Der zweite optische Strahlengang 112_2 verläuft somit an dem zweiten Abschirmelement 120_2 vorbei bzw. um dieses herum.

Ferner kann die Bildaufnahmevorrichtung 100 eine Abschirmung 122 aufweisen, die die drei Spiegel 104_1 bis 104_3 und die drei Kameras 106_1 bis 106_3 einschließt bzw. umfasst und an Seiten des Szintillators 102 angrenzt.

Die Abschirmung 122 kann dabei so geformt sein, dass ein Abschnitt 124 der Abschirmung zwischen Szintillator und der dritten Kamera 106_3 angeordnet ist, so dass die dritte Kamera 106_3 in Bezug auf den Szintillator vollständig hinter dem Abschnitt 124 der Abschirmung angeordnet ist.

Der Abschnitt 124 der Abschirmung 122 und die Abschirmelemente 120_1 und 120_2 können dabei die gleiche oder eine ähnliche Form aufweisen. Wie in Fig. 5 zu erkennen ist, können das erste Abschirmelement 120_1, das zweite Abschirmelement 120_2 und der Abschnitt 124 der Abschirmung 122 I-förmig sein.

Ferner können der Abschnitt 124 der Abschirmung 122 und die Abschirmelemente 120_1 und 120_2 gleich oder ähnlich (in Bezug zueinander bzw. in Bezug auf den Szintillator oder die jeweilige Kamera) angeordnet sein.

Des Weiteren kann die Bildaufnahmevorrichtung 100 drei Strahlenschutzgläser 126_1 bis 126_3 aufweisen.

Das erste Strahlenschutzglas 126_1 kann in dem ersten optischen Strahlengang 112_1 zwischen der ersten Kamera 106_1 und dem ersten Spiegel 104_1 angeordnet sein, so dass die erste Kamera in Bezug auf den ersten optischen Strahlengang 112_1 hinter dem ersten Strahlenschutzglas 126_1 angeordnet ist. Ferner kann das erste Strahlenschutzglas 126_1 zwischen dem ersten Abschirmelement 120_1 und der Abschirmung 122 angeordnet sein, so dass das erste Strahlenschutzglas 126_1 den Bereich zwischen dem ersten Abschirmelement 120_1 und der Abschirmung 122 (vollständig) ausfüllt.

Das zweite Strahlenschutzglas 126_2 kann in dem zweiten optischen Strahlengang 112_2 zwischen der zweiten Kamera 106_2 und dem zweiten Spiegel 104_2 angeordnet sein, so dass die zweite Kamera in Bezug auf den zweiten optischen Strahlengang 112_2 hinter dem zweiten Strahlenschutzglas 126_2 angeordnet ist. Ferner kann das zweite Strahlenschutzglas 126_2 zwischen dem ersten Abschirmelement 120_2 und dem zweiten Abschirmelement 120_2 angeordnet sein, so dass das zweite Strahlenschutzglas 126_1 den Bereich zwischen dem ersten Abschirmelement 120_2 und dem zweiten Abschirmelement 120_2 (vollständig) ausfüllt.

Das dritte Strahlenschutzglas 126_3 kann in dem dritten optischen Strahlengang 112_3 zwischen der dritten Kamera 106_3 und dem dritten Spiegel 104_3 angeordnet sein, so dass die dritte Kamera 106_3 in Bezug auf den dritten optischen Strahlengang 112_3 hinter dem dritten Strahlenschutzglas 126_3 angeordnet ist. Ferner kann das dritte Strahlenschutzglas 126_3 zwischen dem zweiten Abschirmelement 120_2 und der Abschirmung 122 angeordnet sein, so dass das dritte Strahlenschutzglas 126_3 den Bereich zwischen dem zweiten Abschirmelement 120_2 und der Abschirmung 122 (vollständig) ausfüllt.

Fig. 6 zeigt eine schematische Ansicht einer Bildaufnahmevorrichtung 100 mit drei Kameras 106_1 bis 106_3, die unter Scheimpflug-Bedingungen über jeweils einen Spiegel 104_1 bis 104_3 einen Abschnitt des Szintillators 102 aufnehmen. Mit anderen Worten, Fig. 6 zeigt eine optische Abbildung unter Scheimpflug-Bedingung, d.h. bei der der Zentralstrahl der Kameras schräg zum Szintillator verläuft. Es sei darauf hingewiesen, dass, obwohl in Fig. 6 nur drei Kameras gezeigt sind, denkrecht zur Zeichenebene und in horizontaler Richtung beliebig viele Kameras in Reihen angeordnet sein können.

Verglichen mit der in Fig. 5 gezeigten Bildaufnahmevorrichtung, erfolgt bei der Bildaufnahmevorrichtung 100 gemäß Fig. 6 die Aufnahme des Szintillators 102 unter Scheimpflug-Bedingungen d.h. bei der der Zentralstrahl der Kameras schräg zum Szintillator verläuft.

Hierzu werden die Optiken der drei Kameras 106_1 bis 106_3 unter Scheimpflug-Bedingung angeordnet. Ferner sind die drei Spiegel 104_1 bis 104_3 und die drei Kameras 106_1 bis 106_3 so angeordnet, dass die drei Kameras 106_1 bis 106_3 die jeweiligen Abschnitte 110_1 bis 110_3 des Szintillators über die jeweiligen Spiegel 104_1 bis 104_3 jeweils unter Scheimpflug-Bedingungen (d.h. bei der der Zentralstrahl der Kameras schräg zum Szintillator verläuft) aufnehmen.

Die Scheimpflug-Bedingung wird in der Regel angewandt zur optischen Abbildung von zueinander verkippten Objekt- und Bildebenen mittels Objektiv. Die Scheimpflug-Regel besagt, dass sich die Objekt-, die Objektiv- und die Bildebene in einer gemeinsamen Gerade schneiden müssen, um eine scharfe Abbildung der Objektebene auf eine dazu schräg stehenden Bildebene zu erreichen.

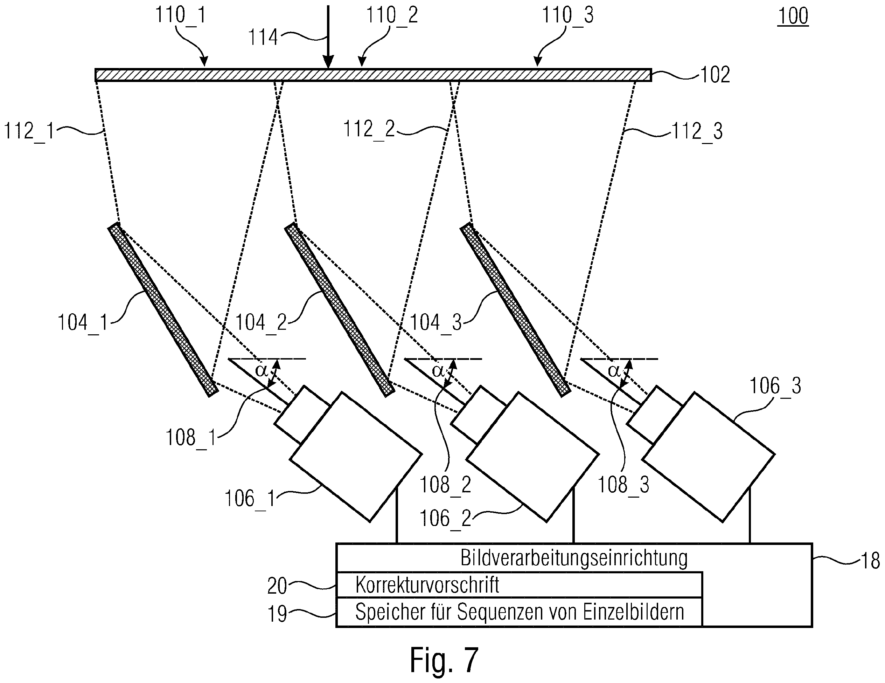

Im Fall der "Scheimpflug-Anordnung" ist im Allgemeinen eine geometrische Umrechnung der Trapez-förmigen Pixel und Kacheln in ein kartesisches System notwendig, wie dies weiter unten u.a. anhand von Fig. 7 erläutert wird.

Durch die in Fig. 6 gezeigte Anordnung der drei Kameras 106_1 bis 106_3 ist es möglich, die drei Kameras 106_1 bis 106_3 hinter dem Szintillator 102 anzuordnen, oder mit anderen Worten, hinter dem Szintillator 102 in Richtung der auf den Schirm 102 treffenden Strahlung (angedeutet durch den Pfeil 114 in Fig. 6). Für den Fall, dass die Strahlung senkrecht auf den Szintillator trifft, würde eine Projektion des Szintillators 102 entlang einer Schirmnormalen somit auf die drei Kameras 106_1 bis 106_3 treffen.

Ferner ist es durch die in Fig. 6 gezeigte Anordnung der drei Kameras 106_1 bis 106_3 möglich, die Abschirmelemente 120_1 und 120_2 zu vergrößern z.B. L-förmig auszuführen, wodurch die Abschirmung der ersten Kamera 106_1 und der zweiten Kamera 106_2 weiter verbessert werden kann. In entsprechender Weise kann auch der Abschnitt 124 der Abschirmung angepasst werden, wodurch auch die Abschirmung der dritten Kamera 106_3 verbessert werden kann.

Die erfindungsgemäße Verbesserung gegenüber der in der DE 103 01 941 gezeigten Vorrichtung wird im Wesentlichen dadurch erreicht, dass die optische Abbildung eines Szintillators mittels eines Kamera-Modul-Arrays über jeweils einzelne gekippte Spiegel und einer entsprechenden Schrägstellung der Kamera-Module realisiert wird. Die Abschirmung der optischen Kameras und der Kamera-Objektive gegen die im Szintillator nicht absorbierte eintreffende Röntgenstrahlung kann im Wesentlichen dadurch erreicht werden, dass zwischen Szintillator und optischen Kameras jeweils ein Absorber mit in Richtung der einfallenden Röntgenstrahlung großer Dicke gebracht wird. Sekundäre Streustrahlung von Spiegel und Absorber kann durch die Verwendung eines speziellen Glasmaterials, das für optisches Licht transparent ist, jedoch Röntgenstrahlung absorbieren kann (z.B. Bleiglas), das kurz vor den Kamera-Objektiven eingebracht werden kann, weitgehend reduziert werden.

Für die Realisierung dieser Anordnung gibt es prinzipiell zwei bevorzugte Ausführungsbeispiele, wie im Folgenden ausgeführt wird.

Gemäß einem ersten Ausführungsbeispiel können die Kamera-Module schräg angeordnet sein und jeweils über einen schräg stehenden Spiegel senkrecht auf den Szintillator blicken (siehe Fig. 5). Dies hat den Vorteil, dass die jeweiligen Kamera-Module einen rechteckigen Ausschnitt des Szintillators abbilden, so dass im Wesentlichen die einem Bildelement (Pixel) entsprechenden Bereiche des Szintillators rechtwinklig und im speziellen quadratisch sind. Zu beachten ist hierbei, dass die für die Abbildung verwendeten Spiegel in diesem Fall direkt in Einstrahlrichtung der eintreffenden Röntgenstrahlung platziert sind, d.h. im Szintillator nicht absorbierte Strahlung kann auf die Spiegel fallen, was zu sekundärer Streustrahlung führen kann.

Gemäß einem zweiten Ausführungsbeispiel können die Kamera-Module schräg angeordnet sein und jeweils unter schrägem Winkel über entsprechend verkippte Spiegel auf den jeweilige Szintillator-Ausschnitt blicken (siehe Fig. 6). Dies hat den Vorteil, dass auch die Spiegel durch zusätzliche Absorber von der primären Röntgenstrahlung abgeschirmt werden können, so dass die entstehende sekundäre Streustrahlung reduziert wird. In diesem Fall sind die abgebildeten Szintillator-Ausschnitte und die Pixel-Ausschnitte gleichschenklig Trapez-förmig. Weiterhin ist die Pixel-Auflösung auf dem Szintillator nicht konstant entsprechend der Trapez-förmigen Verzeichnung der Szintillator-Ausschnitte. Für eine Kachelung einer Vielzahl von Kamera-Modulen in beide Raumrichtungen des Szintillators, d.h. in einem 2-dimensionalem Array, wird erfindungsgemäß vorgeschlagen, dass die Verkippung der Betrachtungsrichtung alternierend zwischen aufeinanderfolgenden Kamera-Modul-Reihen realisiert wird. Dies hat den Vorteil, dass sich die Trapez-förmigen Szintillator-Ausschnitte dicht aneinanderfügen lassen, so dass kein ungleichmäßiger Überlappungsbereich zwischen zwei benachbarten Modul-Reihen auftritt. Für diesen Fall der Abbildung des Szintillators und schrägem Betrachtungswinkel ist es notwendig, eine Objekt-Objektiv-Bild-Geometrie der einzelnen Kamera-Module entsprechend der bekannten Scheimpflug-Bedingung zu realisieren, so dass eine im gesamten Bildbereich scharfe Abbildung des Szintillators auf den Bildsensor des jeweiligen Kamera-Moduls erreicht wird, was sonst ohne Berücksichtigung der Scheimpflug-Bedingung nicht gewährleistet ist. Die Scheimpflug-Bedingung wird in diesem Fall dadurch erreicht, dass die Normale des Bildsensors gegenüber der optischen Achse der Abbildung verkippt wird, so dass sich die Ebenen von Bildsensor, Objektiv und Szintillator in einer gemeinsamen Gerade schneiden.

Bei der vorliegenden Erfindung wird ein Szintillatorschirm in der Weise auf mehrere optische Kameras abgebildet, dass nur das vom Szintillator ausgehende sichtbare Licht auf die optischen Kameras fällt, ggf. durch den Szintillator hindurch tretende Röntgenstrahlung jedoch keine Strahlenschäden verursachen kann.

Wie in Fig. 5 und Fig. 6 gezeigt, gelangt das von einer Teilfläche des Szintillators ausgehende sichtbare Licht über jeweils einen Spiegel zur optischen Kamera. Die Kamera selbst befindet sich hinter einem Absorber aus z.B. Blei oder Wolfram, so dass für Röntgenstrahlung kein direkter Weg zur Kamera existiert.

An den Spiegeln oder Absorber gestreute Röntgenstrahlung kann dennoch auf die Kamera gelangen und dort Strahlungsschäden verursachen, auch wenn die Intensität der gestreuten Röntgenstrahlung erheblich geringer ist als die auf die Kamera eintreffende Röntgenstrahlung. Aus diesem Grund wird an den Stellen, wo der Absorber für die optische Abbildung unterbrochen werden muss, ein spezielles Glas angeordnet ist, das für sichtbares Licht transparent ist, Röntgenstrahlung jedoch stark absorbiert (z.B. Bleiglas).

Durch zusätzliche Absorber oberhalb der Spiegel, wie in Fig. 6 gezeigt, wird der integrale Materialquerschnitt der Abschirmung für die optischen Kameras in Röntgenstrahlrichtung weiter erhöht, so dass der Detektor hierdurch für deutlich höhere Röntgenenergien ohne Strahlenschäden eingesetzt werden kann.

Wie in Fig. 4 bis 6 gezeigt ist können die Kameras (Einzelkameras) 106_1 bis 106_n in einem Array angeordnet werden, wobei jede der Kameras 106_1 bis 106_n eine Teilfläche des abzubildenden Schirms abbildet.

Ferner können die Mehrzahl von Kameras 106_1 bis 106_n in einem zweidimensionalen Array angeordnet werden, wobei jede der Kameras 106_1 bis 106_n eine Teilfläche des abzubildenden Schirms abbildet, wobei sich die Teilflächen überlappen können.

Jeder der Mehrzahl von Kameras 106_1 bis 106_n kann ein Einzelbild des jeweiligen Teilbereichs des abzubildenden Schirms liefern.

Bei Ausführungsbeispielen kann eine Korrektur der digitalen Einzelbilder der Mehrzahl von Kameras 106_1 bis 106_n bei einer Auflösung durchgeführt werden, die höher als die Auflösung ist, die letztendlich die optische Aufnahme der Kamera haben soll. Dadurch können die Kameras 106_1 bis 106_n sowohl eine hohe Lichtempfindlichkeit liefern als auch gleichzeitig Ausgangsbilder lieferen, die für eine automatische Bildweiterverarbeitung geeignet sind. Dieses Konzept ist einerseits dahingehend vorteilhaft, dass normal erhältliche Einzelkameras arraymäßig angeordnet werden können, ohne dass bestimmte Modifikationen an den Kameras beispielsweise zur Auflösungsreduktion, vorgenommen werden müssen. Das durch die Einzelkameras erhaltene Array von Einzelbildern kann dann auf einer höheren Auflösung, die durch den Einsatz üblicher Einzelkameras ohnehin erhalten wird, bereits einer Korrekturverarbeitung unterzogen werden, um die durch die Einzelkameras erhaltenen Einzelbilder untereinander zu justieren und gegebenenfalls vorhandene Überlappungsbereiche zu eliminieren. Aus dem Ergebnis der Korrektur einschließlich einer gegebenenfalls vorhandenen Überlappungsbereicheliminierung kann dann ein Gesamtbild mit einer für die Korrektur verwendeten Auflösung erhalten werden, die höher als die letztendlich benötigte Auflösung ist. Dieses Gesamtbild mit hoher Auflösung kann durchaus Kanten an den Grenzen der einzelnen Teilbilder aufweisen.

Zur Kanten- und Auflösungsreduktion kann dann eine Nachverarbeitung des Gesamtbilds dahingehend durchgeführt, dass benachbarte Pixel beispielsweise durch Addition miteinander kombiniert werden. Die Anzahl von miteinander kombinierten Pixeln hängt von dem Verhältnis der Korrekturauflösung zur letztendlich benötigten Gesamtauflösung ab und kann von Fall zu Fall variieren. Die Zusammenfassung benachbarter Pixel nach der Korrektur führt jedoch dazu, dass die noch im Bild hoher Auflösung sichtbaren Kanten bei Übergang von einem Einzelbild zu einem anderen Einzelbild unsichtbar werden, so dass schließlich ein Gesamtbild mit einer erwünschten Gesamtauflösung erhalten wird, das in seiner Datenmenge gut handhabbar ist, und das ferner keine oder nur kaum sichtbare Kanten mehr an der Grenze von einem Teilbild zum nächsten Teilbild hat. Das letztendlich erhaltene Gesamtbild ist somit für eine nachfolgende Bildverarbeitung aufgrund der Kantenfreiheit einerseits und der reduzierten Datenmenge andererseits gut geeignet.

Als Einzelkameras 106_1 bis 106_n können Kameras eingesetzt werden, die die Möglichkeit des sogenannten "Binnings" erlauben, wie z.B. CCD-Kameras. Beispielsweise kann bei CCD-Kameras bereits vor oder während des Auslesens des CCD-Sensors eine gewissermaßen analoge Ladungsaddition benachbarter Zeilen bzw. Spalten durchgeführt werden, die besonders günstig ist, da diese Addition nicht durch ein elektronisches Rauschen beeinträchtigt wird, das auftreten würde, wenn das Binning nach Auslesen und Analog/Digital-Wandeln digital durchgeführt werden würde.

Ein solches digitales Kombinieren benachbarter Pixel kann lediglich nach Anwendung der Korrekturvorschriften für die einzelnen Einzelbilder ausgeführt werden, um die letztendliche Auflösungsreduktion und Kanteneliminierung zu erreichen.

Es können somit übliche kleinflächige CCD-Kameras mit einer nur gering verkleinernden Linsenoptik eingesetzt werden, so dass im Vergleich zum Einsatz einer einzigen CCD-Kamera mit stark verkleinernder Linsenoptik zwei Empfindlichkeitsvorteile erreicht werden. Zum einen wird der Lichtverlust in der nur schwach verkleinernden Linsenoptik wesentlich geringer sein als in der stark verkleinernden Linsenoptik. Zum anderen wird ein zusätzlicher Empfindlichkeitsvorteil durch das analoge Binning bzw. durch die digitale Kombination benachbarter Pixel nach der Korrektur erhalten.

Aufgrund der Einsatzmöglichkeit von CCD-Sensoren oder auch nur CMOS-Bildsensoren werden Bildwiederholfrequenzen für großflächige Aufnahmen ermöglicht, die bis zu einem Faktor 1000 höher sind als die Wiederholfrequenzen, die mit amorphen Siliziumdetektoren für ähnliche Bildformate erreicht werden können.

Zur Korrektur bzw. Justage der Einzelbilder können für jede Einzelkamera 106_1 bis 106_n eigens bestimmte Korrekturvorschriften verwendet werden. Diese Korrekturvorschriften müssen jedoch nur einmal, beispielsweise vor der Auslieferung der Kamera, bestimmt werden und bleiben dann, wenn die Justage der einzelnen Kameras nicht verändert wird, immer gleich. Solche Korrekturvorschriften beispielsweise in der Form von Nachschlagtabellen, etc. können ohne weiteres fest verdrahtet werden und allgemein gesagt sehr effizient beispielsweise mittels eines DSP ausgeführt werden, da es sich lediglich um einfache Pixelverschiebungen/Sortierungen handelt, die im Hinblick auf ihren benötigten Rechenaufwand unproblematisch sind.

Wie bereits erwähnt wurde, können die Kameras 106_1 bis 106_n im Strahlengang der Röntgenstrahlen hinter der Szintillatorschicht 102 platziert werden, um kurzbrennweitige Objektive einsetzen zu können, die mit höherer Lichtstärke verfügbar sind als die ansonsten erforderlichen langbrennweitigen Objektive. Solche langbrennweitigen Objektive werden dann benötigt, wenn eine Kamera nicht im Strahlengang der Röntgenstrahlen plaziert wird, sondern unter Verwendung einer Spiegeloptik außerhalb der Röntgenstrahlen, um die optische Kamera vor den Röntgenstrahlen zu schützen.

Zum Schutz der Kameras vor Röntgenstrahlen, die durch den Szintillatorschirm hindurchtreten, können zusätzlich Schutzscheiben 126_1 bis 126_n beispielsweise aus Bleiglas eingesetzt werden, die ferner beheizbar ausgeführt sein können, um Beeinträchtigungen des Bleiglases durch Röntgenstrahlen, insbesondere härtere Röntgenstrahlung, entgegenzuwirken.

Fig. 7 zeigt eine schematische Ansicht einer Bildaufnahmevorrichtung 100 gemäß einem Ausführungsbeispiel. Verglichen mit der in Fig. 4 gezeigten Bildaufnahmevorrichtung 100, weist die in Fig. 7 gezeigte Bildaufnahmevorrichtung 100 zusätzlich eine Bildverarbeitungseinrichtung 18 zum Bearbeiten der digitalen Einzelbilder des Arrays von optischen Einzelkameras 106_1 bis 106_n auf, wobei die Bildverarbeitungseinrichtung 18 ausgebildet ist, um die optische Aufnahme des Schirms 102 mit der vorbestimmten Gesamtauflösung zu erzeugen.

Wie bereits erwähnt wurde, kann für die optische Aufnahme des Schirms 102 eine vorbestimmte Gesamtauflösung vorgesehen sein, wobei jede der Mehrzahl von Kameras 106_1 bis 106_n ausgebildet sein kann, um den jeweiligen Abschnitt des Schirms 102, der eine Teilfläche der Schirmfläche umfasst, mit einer Einzelauflösung aufzunehmen, die höher als die Gesamtauflösung ist.

Die Bildverarbeitungseinrichtung 18 kann ausgebildet sein, um die Einzelbilder der Mehrzahl von Kameras zu einem Gesamtbild zu verschmelzen, dass die optische Aufnahme des Schirms mit der vorbestimmten Gesamtauflösung repräsentiert.

Ferner kann die Bildverarbeitungseinrichtung 18 ausgebildet sein, um jedes Pixel des Gesamtbildes durch Gewichtetes Aufsummieren örtlich entsprechender Pixel der Einzelbilder zu berechnen.

Das gewichtete Aufsummieren kann beispielsweise zweistufig erfolgen, wobei zunächst Pixel der Einzelbilder zu einem hochauflösenden Gesamtbild zusammengefasst werden und anschließend ein Binning zur Gesamtauflösung durchgeführt wird. Alternativ kann das gewichtete Aufsummieren auch einstufig erfolgen.

Die örtlich entsprechenden Pixel der Einzelbilder können die Pixel der Einzelbilder sein, die das jeweilige Pixel des Gesamtbilds betrachten bzw. ergeben.

Durch die Auflösungsreduktion kann der Tiefpassfiltereffekt verringert werden. Ferner ist es möglich Verzeichnungen, Lagefehler, Empfindlichkeitsschwankungen der Bildsensoren der Mehrzahl von Kameras und Verschmelzung der Randbereiche der Mehrzahl von Kameras zu korrigieren.

Wie oben bereits beschrieben wurde, können Aufnahmerichtungen der Kameras 106_1 bis 106_n im Wesentlichen parallel zueinander verlaufen. Dabei ist jedoch zu berücksichtigen, dass durch Toleranzen (z.B. Montagetoleranzen bzw. Ausrichtungstoleranzen der Kameras 106_1 bis 106_n oder Fertigungstoleranzen der Kameras 106_1 bis 106_n) die Aufnahmerichtungen der Kameras 106_1 bis 106_n leicht voneinander abweichen bzw. sich unterscheiden können. Auch diese Abweichungen können durch die Bildverarbeitungseinrichtung korrigiert werden.

Ferner kann die Bildverarbeitungseinrichtung 18 wirksam sein, um die digitalen Einzelbilder einer Korrektur zu unterziehen, um Ausrichtungsungenauigkeiten und/oder Parameterschwankungen in dem Array von optischen Einzelkameras 106_1 bis 106_n zu reduzieren und vorzugsweise komplett zu eliminieren. Für die Korrektur eines Einzelbildes wird eine bei einer Kalibrierung, die einer Aufnahme vorausgeht, bestimmte Korrekturvorschrift 20 verwendet, die typischerweise in der Bildverarbeitungseinrichtung 18 auf einem geeigneten Speichermedium abgespeichert ist oder sogar fest verdrahtet ist. Die Korrektur mit der bei der Kalibrierung bestimmten Korrekturvorschrift kann bei einer Korrekturauflösung stattfinden, die höher als die vorbestimmte Gesamtauflösung der am Ende gewünschten optischen Gesamtaufnahme ist, und die niedriger oder gleich der Einzelauflösung ist, mit der die optischen Einzelkameras 106_1 bis 106_n die Einzelbilder liefern. Die Bildverarbeitungseinrichtung 18 kann schließlich wirksam sein, um korrigierte Einzelbilder oder ein korrigiertes Gesamtbild zu erhalten. Das Zusammensetzen der Einzelbilder zu dem Gesamtbild kann somit nach der Korrektur der Einzelbilder mit den für jedes Einzelbild, d.h. für jede Einzelkamera 106_1 bis 106_n bestimmten Korrekturvorschrift 20 vor dem abschließenden Pixelkombinationsschritt stattfinden oder nach dem abschließenden Pixelkombinationsschritt. Aus Effizienz- und Qualitätsgründen wird es jedoch bevorzugt, zunächst das Gesamtbild mit der hohen optischen Auflösung nach der Korrektur der Einzelbilder zusammenzusetzen und dann anhand des zusammengesetzten Gesamtbildes benachbarte Pixel zu addieren, um die optische Aufnahme mit der vorbestimmten Gesamtauflösung zu erhalten. Der abschließende Schritt der Kombination benachbarter Pixel wird somit durchgeführt, um das Gesamtbild, das mit der hohen Korrekturauflösung vorliegt, auf die niedrigere gewünschte vorbestimmte Gesamtauflösung zu bringen. Dieser abschließende Schritt hat, da er bereits mit korrigierten Einzelbildern vorgenommen wird, den Vorteil, dass eventuelle Artefakte an Grenzen der korrigierten Einzelbilder verwischt und damit unkenntlich gemacht werden, und dass ferner eine Auflösungsreduktion stattfindet, um kein Gesamtbild mit einer zu hohen Auflösung zu erhalten. Bilder mit zu hoher Auflösung sind für eine Weiterverarbeitung aufgrund ihrer hohen Datenmenge unhandlich. Außerdem wird für viele Anwendungen eine extrem hohe Auflösung nicht benötigt, so dass ohne weiteres eine Auflösungsreduktion stattfinden kann.

Bei Ausführungsbeispielen wird ausgenutzt, dass durch den Einsatz mehrerer Kameras 106_1 bis 106_n die zur Verfügung stehenden Bildelemente (Pixel) proportional zur Anzahl der Kameras 106_1 bis 106_n wächst. Häufig wird jedoch, wie es ausgeführt worden ist, keine größere Pixelanzahl benötigt, als diejenige, die eine einzige Kamera liefern würde, wie dies im Folgenden beispielhaft ausgeführt wird.

Werden nunmehr beispielsweise vier optische Kameras eingesetzt, können in diesem Fall jeweils vier Pixel aufaddiert werden. Besonders vorteilhaft ist dies, wenn die Ladung bereits auf dem Sensor aufaddiert werden kann, wie es bei CCD-Sensoren durch das sogenannte Binning geschieht. In diesem Fall muss die Ladung nur einmal elektronisch ausgelesen werden, und es entsteht somit nur einmal das durch diesen Vorgang erzeugte elektronische Rauschen, so dass das insgesamte Signal/Rausch-Verhältnis besser ist, als wenn jedes Pixel einzeln ausgelesen wird und digital addiert wird. Im vorliegenden beispielhaften Fall der 4 × 4 Situation kann die Korrektur entweder bei der höchsten Auflösung stattfinden, also ohne dass ein Binning durchgeführt worden ist. Alternativ könnte jedoch beispielhaft bereits ein 2 × 2-Binning durchgeführt werden, um die Korrektur bei einer vierfachen Auflösung bezüglich der gewünschten Gesamtauflösung durchzuführen. Hierbei wäre dann ein abschließender Auflösungsreduktionsschritt durch digitales Kombinieren benachbarter Pixel beispielsweise durch Addition möglich, wobei immer 2 × 2 Pixel, also vier benachbarte Pixel zusammengefasst werden, und zwar gemäß einer bestimmten Kombinationsvorschrift, die entweder überlappend zusammenfassen kann, oder aneinander angrenzend zusammenfassen kann oder auf irgendeine andere Art und Weise eine Pixelkombination benachbarter Pixel erreicht. Damit ist eine optimal genaue Korrektur bei hoher Auflösung möglich und ist gleichzeitig aufgrund der Auflösungsreduktion nach der Korrektur eine Unterdrückung von Artefakten wegen Einzelbilder-Kanten erreicht.

Wie in Fig. 7 zu erkennen ist, kann sich hinter dem Szintillatorschirm 102 das Array von Kameras 106_1 bis 106_n befinden, wobei jede der Kameras 106_1 bis 106_n jeweils einen Teil des Szintillatorschirms 102 abbildet. Für spezielle Aufgaben, bei denen gewöhnlich eine Zeilenkamera eingesetzt wird, degeneriert das Array von optischen Einzelkameras 106_1 bis 106_n zu einem eindimensionalen Array, dass eine lineare Anordnung optischer Einzelkameras umfasst. Die von den einzelnen optischen Kameras abgebildeten Bereiche können entweder unmittelbar aneinander angrenzen oder überlappen sich geringfügig, um den Justage-Aufwand der typischerweise auf mechanischer Seite anfallen würde, zu reduzieren.

Haben die Teilbilder bzw. Einzelbilder eine Überlappung, so kann eine elektronische Korrektur durchgeführt werden. Bei Überlappung der Teilbilder wird insbesondere der in beiden Kameras 106_1 bis 106_n abgebildete Bereich nach dem Auslesen der einzelnen optischen Kameras 106_1 bis 106_n verworfen, und die optische Gesamtaufnahme wird nach der Korrektur, die dann auch ein Verwerfen des Überlappungsbereichs umfasst, aus den korrigierten und einer Verwerfung unterzogenen Einzelbildern zusammengesetzt und Auflösungsreduziert.

Obwohl oben Ausführungsbeispiele beschrieben wurden, bei denen die Kameras 106_1 bis 106_n in einem zweidimensionalen Gitter angeordnet sind, das kongruent zu einem Gitter ist, entsprechend dem die durch die Kameras aufgenommenen Abschnitte bzw. Teilflächen des Schirms angeordnet sind, sei darauf hingewiesen, dass dies nicht erforderlich sind. Vielmehr können die Kameras 106_1 bis 106_n auch in einem zweidimensionalen Gitter angeordnet sein, das nicht kongruent zu einem Gitter ist, entsprechend dem die durch die Kameras aufgenommenen Abschnitte bzw. Teilflächen des Schirms angeordnet sind.

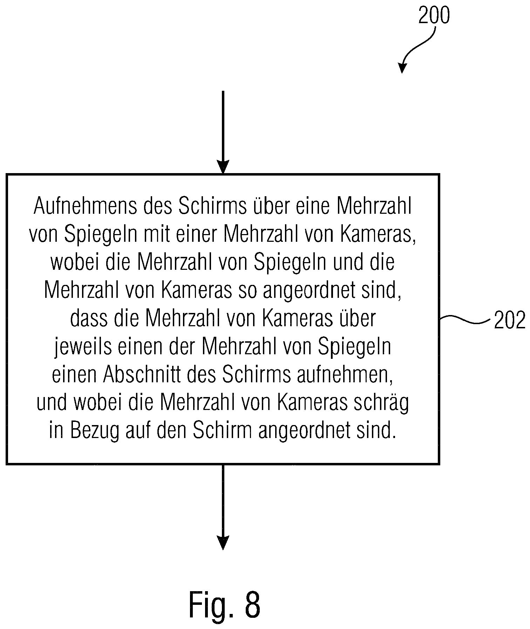

Fig. 8 zeigt ein Flussdiagramm eines Verfahrens 200 zum Aufnehmen eines Schirms. Das Verfahren 200 umfasst einen Schritt 202 des Aufnehmens des Schirms über eine Mehrzahl von Spiegeln mit einer Mehrzahl von Kameras, wobei die Mehrzahl von Spiegeln und die Mehrzahl von Kameras so angeordnet sind, dass die Mehrzahl von Kameras über jeweils einen der Mehrzahl von Spiegeln einen Abschnitt des Schirms aufnimmt, und wobei die Mehrzahl von Kameras schräg in Bezug auf den Schirm angeordnet ist.

Weitere Ausführungsbeispiele schaffen eine Vorrichtung zur Abbildung eines Schirms (Szintillator) mittels einer Vielzahl von Kamera-Modulen, dadurch gekennzeichnet, dass die Abbildung erreicht wird durch eine Aneinanderreihung von Kamera-Modulen, die schräg angeordnet sind und über einen entsprechend gekippten Spiegel auf den Schirm blicken.

Bei den weiteren Ausführungsbeispielen kann ein dickes strahlungsabsorbierendes Material (Absorber) zwischen Szintillator und Kameras angebracht werden, so dass eine Strahlenschutzwirkung bei hohen Röntgenenergien (z.B. > 200 keV, oder > 450 keV) erreicht werden kann.

Bei den weiteren Ausführungsbeispielen kann die Abbildung des Szintillators parallel zur Normalen des Szintillators geschehen. Ein Zentralstrahl des optischen Strahlengangs zwischen dem jeweiligen Abschnitt des Schirms und dem jeweiligen Spiegel kann hierbei also parallel zu der Normalen des Schirms verlaufen.

Bei den weiteren Ausführungsbeispielen kann die Abbildung des Szintillators schräg zur Normalen des Szintillators geschehen. Ein Zentralstrahl des optischen Strahlengangs zwischen dem jeweiligen Abschnitt des Schirms und dem jeweiligen Spiegel kann hierbei also schräg zu der Normalen des Schirms verlaufen.

Bei den weiteren Ausführungsbeispielen kann eine scharfe Abbildung des Szintillators mittels Scheimpflug-Bedingung erreicht werden.

Bei den weiteren Ausführungsbeispielen kann ein strahlungsabsorbierendes Glas zwischen Spiegel und Objektiv gebracht werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Der Datenträger, das digitale Speichermedium oder das computerlesbare Medium sind typischerweise gegenständlich und/oder nicht-vergänglich bzw. nichtvorübergehend.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die hierin beschriebenen Vorrichtungen können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Vorrichtungen, oder jedwede Komponenten der hierin beschriebenen Vorrichtungen können zumindest teilweise in Hardware und/oder in Software (Computerprogramm) implementiert sein.

Die hierin beschriebenen Verfahren können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Verfahren, oder jedwede Komponenten der hierin beschriebenen Verfahren können zumindest teilweise durch Hardware und/oder durch Software ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Bildaufnahmevorrichtung (100), mit folgenden Merkmalen:
einem Schirm (102);
einer Mehrzahl von Spiegeln (104_1-104_n); und
einer Mehrzahl von Kameras (106_1-106_n);
wobei die Mehrzahl von Spiegeln (104_1-104_n) und die Mehrzahl von Kameras (106_1-106_n) so angeordnet sind, dass eine erste Kamera (106_1) der Mehrzahl von Kameras (106_1-106_n) über einen ersten Spiegel (104_1) der Mehrzahl von Spiegeln (104_1-104_n) einen ersten Abschnitt (110_1) des Schirms (102), eine zweite Kamera (106_2) der Mehrzahl von Kameras (106_1-106_n) über einen zweiten Spiegel (104_2) der Mehrzahl von Spiegeln (104_1-104_n) einen zweiten Abschnitt (110_2) des Schirms (102), und eine dritte Kamera (106_3) der Mehrzahl von Kameras (106_1-106_n) über einen dritten Spiegel (104_3) der Mehrzahl von Spiegeln (104_1-104_n) einen dritten Abschnitt (110_3) des Schirms (102) aufnimmt;
wobei die Mehrzahl von Kameras (106_1-106_n) schräg in Bezug auf den Schirm (102) angeordnet sind; und
wobei die Mehrzahl von Kameras (106_1-106_n) in Bezug auf den Schirm (102) so angeordnet sind, dass Winkel zwischen Aufnahmerichtungen (108_1-108_n) der Mehrzahl von Kameras (106_1-106_n) und einer Schirmebene zwischen 5° und 85° betragen.

2. Bildaufnahmevorrichtung (100) nach Anspruch 1, wobei die Mehrzahl von Kameras (106_1-106_n) so angeordnet ist, dass Aufnahmerichtungen (108_1-108_n) der Mehrzahl von Kameras (106_1-106_n) parallel zueinander verlaufen.

3. Bildaufnahmevorrichtung (100) nach einem der Ansprüche 1 bis 2, wobei zumindest eine der Mehrzahl von Kameras (106_1-106_n) hinter dem Schirm (102) angeordnet ist.

4. Bildaufnahmevorrichtung (100) nach einem der Ansprüche 1 bis 3, wobei die Bildaufnahmevorrichtung (100) zumindest ein Abschirmelement (120_1,120_2) aufweist, das zwischen dem Schirm (102) und einer der Mehrzahl von Kameras (106_1-106_n) angeordnet ist, so dass die zumindest eine der Mehrzahl von Kameras (106_1-106_n) in Bezug auf den Schirm (102) vollständig hinter dem Abschirmelement (120_1,120_2) angeordnet ist.

5. Bildaufnahmevorrichtung (100) nach Anspruch 4, wobei das zumindest eine Abschirmelement (120_1,120_2) außerhalb eines optischen Strahlengangs (112_1,112_2) angeordnet ist, der zwischen der einen der Mehrzahl von Kameras (106_1-106_n) über den jeweiligen der Mehrzahl von Spiegeln (104_1-104_n) und dem jeweiligen Abschnitt (110_1-110_n) des Schirms verläuft.

6. Bildaufnahmevorrichtung (100) nach einem der Ansprüche 4 bis 5, wobei das zumindest eine Abschirmelement (120_1,120_2) ausgebildet ist, um eine Röntgenenergie von 200 keV oder mehr zu absorbieren.

7. Bildaufnahmevorrichtung (100) nach Anspruch 6, wobei das zumindest eine Abschirmelement (120_1,120_2) L-förmig ist.

8. Bildaufnahmevorrichtung (100) nach Anspruch 6, wobei das zumindest eine Abschirmelement (120_1,120_2) I-förmig ist.

9. Bildaufnahmevorrichtung (100) nach einem der Ansprüche 1 bis 8, wobei die Bildaufnahmevorrichtung (100) eine Abschirmung (122) aufweist, die die Mehrzahl von Spiegeln (104_1-104_n) und die Mehrzahl von Kameras (106_1-106_n) einschließt und an Seiten des Schirms (102) angrenzt.

10. Bildaufnahmevorrichtung (100) nach Anspruch 9, wobei die Abschirmung (122) so geformt ist, dass ein Abschnitt (124) der Abschirmung (122) zwischen dem Schirm (102) und einer der Mehrzahl von Kameras (106_1-106_n) angeordnet ist, so dass die eine der Mehrzahl von Kameras (106_1-106_n) in Bezug auf den Schirm (102) vollständig hinter dem Abschnitt (124) der Abschirmung (122) angeordnet ist.

11. Bildaufnahmevorrichtung (100) nach Anspruch 10, wobei zumindest eine andere der Mehrzahl von Kameras (106_1-106_n) hinter dem zumindest einen Abschirmelement (120_1,120_2) nach einem der Ansprüche 7 bis 9 angeordnet ist.

12. Bildaufnahmevorrichtung (100) nach einem der Ansprüche 1 bis 11, wobei die Bildaufnahmevorrichtung (100) eine Mehrzahl von Strahlenschutzgläsern (126_1-126_n) aufweist, die zwischen der Mehrzahl von Spiegeln (104_1-104_n) und der Mehrzahl von Kameras (106_1-106_n) angeordnet ist.

13. Bildaufnahmevorrichtung (100) nach einem der Ansprüche 1 bis 12, wobei die Mehrzahl von Kameras (106_1-106_n) den jeweiligen Abschnitt des Schirms (102) jeweils über genau einen der Mehrzahl von Spiegeln (104_1-104_n) aufnimmt.

14. Bildaufnahmevorrichtung (100) nach einem der Ansprüche 1 bis 13, wobei eine Anzahl der Spiegel (104_1-104_n) gleich einer Anzahl der Kameras (106_1-106_n) ist.

15. Bildaufnahmevorrichtung (100) nach einem der Ansprüche 1 bis 14, wobei der Schirm (102) ein Szintillator ist.

16. Bildaufnahmevorrichtung (100) nach einem der Ansprüche 1 bis 15, wobei die Mehrzahl von Spiegel (104_1-104_n) und die Mehrzahl von Kameras (106_1-106_n) so angeordnet ist, dass die Mehrzahl von Kameras (106_1-106_n) sich zumindest teilweise unterscheidende Abschnitte (110_1-110_n) des Schirms (102) aufnimmt.

17. Bildaufnahmevorrichtung (100) nach einem der Ansprüche 1 bis 16, wobei die Mehrzahl von Kamera-Optik-Kombinationen (106_1-106_n) eine Scheimpflug-Anordnung aufweist;
wobei die Mehrzahl von Spiegeln (104_1-104_n) und die Mehrzahl von Kameras (106_1-106_n) so angeordnet ist, dass die Mehrzahl von Kameras (106_1-106_n) die jeweiligen Abschnitte (110_1-110_n) des Schirms (102) über die jeweiligen Spiegel (104_1-104_n) jeweils unter Scheimpflug-Bedingungen aufnimmt.

18. Bildaufnahmevorrichtung (100) nach einem der Ansprüche 1 bis 17, wobei eine Abbildung des Schirms (102) parallel zu einer Normalen des Schirms (102) erfolgt.

19. Bildaufnahmevorrichtung (100) nach einem der Ansprüche 1 bis 18, wobei eine Abbildung des Schirms (102) schräg zu einer Normalen des Schirms (102) erfolgt.

20. Bildaufnahmevorrichtung (100) nach einem der Ansprüche 1 bis 19, wobei der Schirm (102) eine Fläche aufweist, und wobei für die optische Aufnahme des Schirms (102) eine vorbestimmte Gesamtauflösung vorgesehen ist, wobei jede der Mehrzahl von Kameras (106_1-106_n) ausgebildet ist, um den jeweiligen Abschnitt des Schirms (102), der eine Teilfläche der Schirmfläche umfasst, mit einer Einzelauflösung aufzunehmen, die höher als die Gesamtauflösung ist.

21. Bildaufnahmevorrichtung (100) nach Anspruch 20, wobei die Bildaufnahmevorrichtung (100) eine Bildverarbeitungseinrichtung (18) zum Bearbeiten von Einzelbildern der Mehrzahl von Kamers (106_1-106_n) aufweist, wobei die Bildverarbeitungseinrichtung (18) ausgebildet ist, um die Einzelbilder der Mehrzahl von Kameras (106_1-106_n) einem Gesamtbild zu verschmelzen, dass die optische Aufnahme des Schirms mit der vorbestimmten Gesamtauflösung repräsentiert.

22. Bildaufnahmevorrichtung (100) nach Anspruch 21, wobei die Bildverarbeitungseinrichtung (18) ausgebildet ist, um jedes Pixel des Gesamtbildes durch gewichtetes Aufsummieren örtlich entsprechender Pixel der digitalen Einzelbilder zu berechnen.

23. Verfahren (200) zum Aufnehmen eines Schirms, aufweisend:
Aufnehmen (202) des Schirms über eine Mehrzahl von Spiegeln mit einer Mehrzahl von Kameras;
wobei die Mehrzahl von Spiegeln und die Mehrzahl von Kameras so angeordnet ist, dass eine erste Kamera (106_1) der Mehrzahl von Kameras (106_1-106_n) über einen ersten Spiegel (104_1) der Mehrzahl von Spiegeln (104_1-104_n) einen ersten Abschnitt (110_1) des Schirms (102), eine zweite Kamera (106_2) der Mehrzahl von Kameras (106_1-106_n) über einen zweiten Spiegel (104_2) der Mehrzahl von Spiegeln (104_1-104_n) einen zweiten Abschnitt (110_2) des Schirms (102), und eine dritte Kamera (106_3) der Mehrzahl von Kameras (106_1-106_n) über einen dritten Spiegel (104_3) der Mehrzahl von Spiegeln (104_1-104_n) einen dritten Abschnitt (110_3) des Schirms (102)aufnimmt;
wobei die Mehrzahl von Kameras schräg in Bezug auf den Schirm angeordnet ist; und
wobei die Mehrzahl von Kameras (106_1-106_n) in Bezug auf den Schirm (102) so angeordnet ist, dass Winkel zwischen Aufnahmerichtungen (108_1-108_n) der Mehrzahl von Kameras (106_1-106_n) und einer Schirmebene zwischen 5° und 85° betragen.

24. Computerprogramm zur Durchführung des Verfahrens nach Anspruch 23.

## Claims

1. An image capturing apparatus (100), comprising:
a screen (102);
a plurality of mirrors (104_1 - 104_n); and
a plurality of cameras (106_1 - 106_n);
wherein the plurality of mirrors (104_1 - 104_n) and the plurality of cameras (106_1 - 106_n) are arranged such that a first camera (106_1) of the plurality of cameras (106_1 - 106_n) captures a first portion (110_1) of the screen (102) via a first mirror (104_1) of the plurality of mirrors (104_1 - 104_n), a second camera (106_2) of the plurality of cameras (106_1 - 106_n) captures a second portion (110_2) of the screen (102) via a second mirror (104_2) of the plurality of mirrors (104_1 -104_n), and a third camera (106_3) of the plurality of cameras (106_1 - 106_n) captures a third portion (110_3) of the screen (102) via a third mirror (104_3) of the plurality of mirrors (104_1 - 104_n);
wherein the plurality of cameras (106_1 - 106_n) is obliquely arranged with respect to the screen (102);
wherein the plurality of cameras (106_1 - 106_n) is arranged with respect to the screen (102) such that angles between capturing directions (108_1 - 108_n) of the plurality of cameras (106_1 - 106_n) and a screen plane are between 5° and 85°.

2. The image capturing apparatus (100) according to claim 1, wherein the plurality of cameras (106_1 - 106_n) is arranged such that the capturing directions (108_1 - 108_n) of the plurality of cameras (106_1 - 106_n) extend in parallel to each other.

3. The image capturing apparatus (100) according to any one of claims 1 to 2, wherein at least one of the plurality of cameras (106_1 - 106_n) is arranged behind the screen (102).

4. The image capturing apparatus (100) according to any one of claims 1 to 3, wherein the image capturing apparatus (100) comprises at least one shielding element (120_1, 120_2) arranged between the screen (102) and one of the plurality of cameras (106_1 - 106_n) so that the at least one of the plurality of cameras (106_1 - 106_n) is entirely arranged behind the shielding element (120_1, 120_2) with respect to the screen (102).

5. The image capturing apparatus (100) according to claim 4, wherein the at least one shielding element (120_1, 120_2) is arranged outside an optical path (112_1, 112_2) extending between the one of the plurality of cameras (106_1 - 106_n) and the respective portion (110_1, 110_n) of the screen via the respective one of the plurality of mirrors (104_1 - 104_n).

6. The image capturing apparatus (100) according to any one of claims 4 to 5, wherein the at least one shielding element (120_1, 120_2) is configured to absorb X-ray energy of 200 keV or more.

7. The image capturing apparatus (100) according to claim 6, wherein the at least one shielding element (120_1, 120_2) is L-shaped.

8. The image capturing apparatus (100) according to claim 6, wherein the at least one shielding element (120_1, 120_2) is I-shaped.

9. The image capturing apparatus (100) according to any one of claims 1 to 8, wherein the image capturing apparatus (100) comprises a shield (122) enclosing the plurality of mirrors (104_1 - 104_n) and the plurality of cameras (106_1 - 106_n) and bordering at sides of the screen (102).

10. The image capturing apparatus (100) according to claim 9, wherein the shield (122) is shaped such that a portion (124) of the shield (122) is arranged between the screen (102) and one of the plurality of cameras (106_1 - 106_n) so that the one of the plurality of cameras (106_1 - 106_n) is entirely arranged behind the portion (124) of the shield (122) with respect to the screen (102).

11. The image capturing apparatus (100) according to claim 10, wherein at least one other of the plurality of cameras (106_1 - 106_n) is arranged behind the at least one shielding element (120_1, 120_2) according to any one of claims 7 to 9.

12. The image capturing apparatus (100) according to any one of claims 1 to 11, wherein the image capturing apparatus (100) comprises a plurality of radiation-protection glasses (126_1 - 126_n) arranged between the plurality of mirrors (104_1 - 104_n) and the plurality of cameras (106_1 - 106_n).

13. The image capturing apparatus (100) according to any one of claims 1 to 12, wherein the plurality of cameras (106_1 - 106_n) captures the respective portion of the screen (102) via exactly one of the multitude of mirrors (104_1 - 104_n), respectively.

14. The image capturing apparatus (100) according to any one of claims 1 to 13, wherein a number of the mirrors (104_1 - 104_n) is equal to a number of the cameras (106_1 - 106_n).

15. The image capturing apparatus (100) according to any one of claims 1 to 14, wherein the screen (102) is a scintillator.

16. The image capturing apparatus (100) according to any one of claims 1 to 15, wherein the plurality of mirrors (104_1 - 104_n) and a plurality of cameras (106_1 - 106_n) are arranged such that the plurality of cameras (106_1, 106_n) captures at least partially differing portions (110_1 - 110_n) of the screen (102).

17. The image capturing apparatus (100) according to any one of claims 1 to 16, wherein the plurality of camera/optic combinations (106_1 - 106_n) comprises a Scheimpflug arrangement;
wherein the plurality of mirrors (104_1 - 104_n) and the plurality of cameras (106_1 - 106_n) are arranged such that the plurality of cameras (106_1 - 106_n) each captures the respective portion (110_1 - 110_n) of the screen (102) under Scheimpflug conditions via the respective mirrors (104_1 - 104_n).

18. The image capturing apparatus (100) according to any one of claims 1 to 17, wherein imaging the screen (102) takes place in parallel to a normal of the screen (102).

19. The image capturing apparatus (100) according to any one of claims 1 to 18, wherein imaging the screen (102) takes place obliquely to a normal of the screen (102).

20. The image capturing apparatus (100) according to any one of claims 1 to 19, wherein the screen (102) comprises an area, and wherein a predetermined total resolution is provided for the optical capturing of the screen (102), wherein each of the plurality of cameras (106_1 - 106_n) is configured to capture the respective portion of the screen (102), which includes a partial area of the screen area, with an individual resolution higher that is higher than the total resolution.

21. The image capturing apparatus (100) according to claim 20, wherein the image capturing apparatus (100) comprises an image processing means (18) for processing individual images of the plurality of cameras (106_1 - 106_n), wherein the image processing means (18) is configured to melt the individual images of the plurality of cameras (106_1 - 106_n) into a total image that represents the optical capturing of the screen with the predetermined total resolution.

22. The image capturing apparatus (100) according to claim 21, wherein the image processing means (18) is configured to compute each pixel of the total image by weighted summing up of locally corresponding pixels of the digital individual images.

23. A method (200) for capturing a screen, comprising:
capturing (202) the screen with a plurality of cameras via a plurality of mirrors;
wherein the plurality of mirrors (104_1 - 104_n) and the plurality of cameras (106_1 - 106_n) are arranged such that a first camera (106_1) of the plurality of cameras (106_1 - 106_n) captures a first portion (110_1) of the screen (102) via a first mirror (104_1) of the plurality of mirrors (104_1 - 104_n), a second camera (106_2) of the plurality of cameras (106_1 - 106_n) captures a second portion (110_2) of the screen (102) via a second mirror (104_2) of the plurality of mirrors (104_1 -104_n), and a third camera (106_3) of the plurality of cameras (106_1 - 106_n) captures a third portion (110_3) of the screen (102) via a third mirror (104_3) of the plurality of mirrors (104_1 - 104_n);
wherein the plurality of cameras is arranged obliquely with respect to the screen; and
wherein the plurality of cameras (106_1 - 106_n) is arranged with respect to the screen (102) such that angles between capturing directions (108_1 - 108_n) of the plurality of cameras (106_1 - 106_n) and a screen plane are between 5° and 85°.

24. A computer program for performing the method according to claim 23.

## Revendications

1. Dispositif de capture d'image (100), aux caractéristiques suivantes:
un écran (102);
une pluralité de miroirs (104_1-104_n); et
une pluralité de caméras (106_1-106_n);
dans lequel la pluralité de miroirs (104_1-104_n) et la pluralité de caméras (106_1-106_n) sont disposés de sorte qu'une première caméra (106_1) de la pluralité de caméras (106_1-106_n) capture, par l'intermédiaire d'un premier miroir (104_1) de la pluralité de miroirs (104_1-104_n), un premier segment (110_1) de l'écran (102), qu'une deuxième caméra (106_2) de la pluralité de caméras (106_1-106_n) capture, par l'intermédiaire d'un deuxième miroir (104_2) de la pluralité de miroirs (104_1-104_n), un deuxième segment (110_2) de l'écran (102), et qu'une troisième caméra (106_3) de la pluralité de caméras (106_1-106_n) capture, par l'intermédiaire d'un troisième miroir (104_3) de la pluralité de miroirs (104_1-104_n), un troisième segment (110_3) de l'écran (102);
dans lequel la pluralité de caméras (106_1-106_n) sont disposées obliquement par rapport à l'écran (102); et
dans lequel la pluralité de caméras (106_1-106_n) sont disposées par rapport à l'écran (102) de sorte que les angles entre les directions de prise de vue (108_1-108_n) de la pluralité de caméras (106_1-106_n) et un plan d'écran soient compris entre 5° et 85°.

2. Dispositif de capture d'image (100) selon la revendication 1, dans lequel la pluralité de caméras (106_1-106_n) sont disposées de sorte que les directions de prise de vue (108_1-108_n) de la pluralité de caméras (106_1-106_n) soient parallèles entre elles.

3. Dispositif de capture d'image (100) selon l'une des revendications 1 à 2, dans lequel au moins une de la pluralité de caméras (106_1-106_n) est disposée derrière l'écran (102).

4. Dispositif de capture d'image (100) selon l'une des revendications 1 à 3, dans lequel le dispositif de capture d'image (100) présente au moins un élément de blindage (120_1, 120_2) qui est disposé entre l'écran (102) et l'une de la pluralité de caméras (106_1-106_n), de sorte que l'au moins une de la pluralité de caméras (106_1-106_n) soit disposée, par rapport à l'écran (102), complètement derrière l'élément de blindage (120_1, 120_2).

5. Dispositif de capture d'image (100) selon la revendication 4, dans lequel l'au moins un élément de blindage (120_1, 120_2) est disposé en-dehors d'un trajet de faisceau optique (112_1, 112_2) qui s'étend entre l'une de la pluralité de caméras (106_1-106_n) par l'intermédiaire de l'un respectif de la pluralité de miroirs (104_1-104_n) et le segment respectif (110_1-110_n) de l'écran.

6. Dispositif de capture d'image (100) selon l'une des revendications 4 à 5, dans lequel l'au moins un élément de blindage (120_1, 120_2) est conçu pour absorber une énergie de rayons X de 200 keV ou plus.

7. Dispositif de capture d'image (100) selon la revendication 6, dans lequel l'au moins un élément de blindage (120_1, 120_2) est en forme de "L".

8. Dispositif de capture d'image (100) selon la revendication 6, dans lequel l'au moins un élément de blindage (120_1, 120_2) est en forme de "I".

9. Dispositif de capture d'image (100) selon l'une quelconque des revendications 1 à 8, dans lequel le dispositif de capture d'image (100) présente un blindage (122) qui contient la pluralité de miroirs (104_1-104_n) et la pluralité de caméras (106_1-106_n) et qui vient se raccorder à des côtés de l'écran (102).

10. Dispositif de capture d'image (100) selon la revendication 9, dans lequel le blindage (122) est formé de sorte qu'un segment (124) du blindage (122) soit disposé entre l'écran (102) et l'une de la pluralité de caméras (106_1-106_n), de sorte que l'une de la pluralité de caméras (106_1-106_n) soit disposée, par rapport à l'écran (102), complètement derrière le segment (124) du blindage (122).

11. Dispositif de capture d'image (100) selon la revendication 10, dans lequel au moins une autre de la pluralité de caméras (106_1-106_n) est disposée derrière l'au moins un élément de blindage (120_1, 120_2) selon l'une des revendications 7 à 9.

12. Dispositif de capture d'image (100) selon l'une des revendications 1 à 11, dans lequel le dispositif de capture d'image (100) présente une pluralité de verres de protection contre les rayons (126_1-126_n) qui sont disposés entre la pluralité de miroirs (104_1-104_n) et la pluralité de caméras (106_1-106_n).

13. Dispositif de capture d'image (100) selon l'une des revendications 1 à 12, dans lequel la pluralité de caméras (106_1-106_n) capture le segment respectif de l'écran (102) chaque fois par l'intermédiaire d'exactement l'un de la pluralité de miroirs (104_1-104_n).

14. Dispositif de capture d'image (100) selon l'une des revendications 1 à 13, dans lequel un nombre de miroirs (104_1-104_n) est égal à un nombre de caméras (106_1-106_n).

15. Dispositif de capture d'image (100) selon l'une des revendications 1 à 14, dans lequel l'écran (102) est un scintillateur.

16. Dispositif de capture d'image (100) selon l'une des revendications 1 à 15, dans lequel la pluralité de miroirs (104_1-104_n) et la pluralité de caméras (106_1-106_n) sont disposés de sorte que la pluralité de caméras (106_1-106_n) capturent des segments au moins partiellement différents (110_1-110_n) de l'écran (102).

17. Dispositif de capture d'image (100) selon l'une des revendications 1 à 16, dans lequel la pluralité de combinaisons caméra-optique (106_1-106_n) présente un aménagement de Scheimpflug;
dans lequel la pluralité de miroirs (104_1-104_n) et la pluralité de caméras (106_1-106_n) sont disposés de sorte que la pluralité de caméras (106_1-106_n) capturent les segments respectifs (110_1-110_n) de l'écran (102) par l'intermédiaire des miroirs respectifs (104_1-104_n) dans les conditions de Scheimpflug.

18. Dispositif de capture d'image (100) selon l'une des revendications 1 à 17, dans lequel une reproduction de l'écran (102) a lieu de manière parallèle à une normale de l'écran (102).

19. Dispositif de capture d'image (100) selon l'une des revendications 1 à 18, dans lequel une reproduction de l'écran (102) a lieu de manière oblique par rapport à une normale de l'écran (102).

20. Dispositif de capture d'image (100) selon l'une des revendications 1 à 19, dans lequel l'écran (102) présente une surface, et dans lequel est prévue, pour la capture optique de l'écran (102), une résolution globale prédéterminée, dans lequel chacune de la pluralité de caméras (106_1-106_n) est conçue pour capturer le segment respectif de l'écran (102), qui comporte une surface partielle de la surface d'écran, avec une résolution individuelle qui est supérieure à la résolution totale.

21. Dispositif de capture d'image (100) selon la revendication 20, dans lequel le dispositif de capture d'image (100) présente un dispositif de traitement d'image (18) destiné à traiter les images individuelles de la pluralité de caméras (106_1-106_n), le dispositif de traitement d'image (18) étant conçu pour fondre les images individuelles de la pluralité de caméras (106_1-106_n) en une image globale qui représente la capture optique de l'écran à la résolution totale prédéterminée.

22. Dispositif de capture d'image (100) selon la revendication 21, dans lequel le dispositif de traitement d'image (18) est conçu pour calculer chaque pixel de l'image globale par addition pondérée des pixels localement correspondants des images individuelles numériques.

23. Procédé (200) permettant de capturer un écran, présentant le fait de:
capturer (202) l'écran, par l'intermédiaire d'une pluralité de miroirs, par une pluralité de caméras;
dans lequel la pluralité de miroirs et la pluralité de caméras sont disposés de sorte qu'une première caméra (106_1) de la pluralité de caméras (106_1-106_n) capture, par l'intermédiaire d'un premier miroir (104_1) de la pluralité de miroirs (104_1-104_n), un premier segment (110_1) de l'écran (102), qu'une deuxième caméra (106_2) de la pluralité de caméras (106_1-106_n) capture, par l'intermédiaire d'un deuxième miroir (104_2) de la pluralité de miroirs (104_1-104_n), un deuxième segment (110_2) de l'écran (102), et qu'une troisième caméra (106_3) de la pluralité de caméras (106_1-106_n) capture, par l'intermédiaire d'un troisième miroir (104_3) de la pluralité de miroirs (104_1-104_n), un troisième segment (110_3) de l'écran (102);
dans lequel la pluralité de caméras sont disposées de manière oblique par rapport à l'écran; et
dans lequel la pluralité de caméras (106_1-106_n) sont disposées, par rapport à l'écran (102), de sorte que les angles entre les directions de prise de vue (108_1-108_n) de la pluralité de caméras (106_1-106_n) et un plan d'écran soient compris entre 5° et 85°.

24. Programme informatique pour mettre en oeuvre le procédé selon la revendication 23.
